# EUROPEAN PATENT APPLICATION

(11) **EP 2 382 972 A1**
(43) Date of publication of application: **02.11.2011**
(21) Application number: 11175664.9
(22) Date of filing: 25.01.2008
(51) Int. Cl.: A61K 31/351, A61K 31/70, A61P 25/00, A61P 25/28

(54) **Methods for preventing and treating neurodegenerative disorders**

(62) Divisional of application: 08701664.8
(71) Applicant: Boehringer Ingelheim International GmbH, 55216 Ingelheim am Rhein (DE)
(72) Inventor: Wienrich, Marion, 64331 WEITERSTADT (DE); Reess, Juergen, 89075 ULM (DE)
(74) Representative: Hammann, Heinz

(57) **Abstract**

The present invention relates to a method for treating, preventing or slowing, delaying or reversing progression of one or more neurodegenerative disorders in a patient in need thereof characterized by administering a glucopyranosyl-substituted benzene derivative, a tautomer, stereoisomer, mixture or salt thereof, as defined in claim 1 to the patient in need thereof.

## Description

### BACKGROUND OF THE INVENTION

The present invention relates to methods for preventing and treating neurodegenerative disorders in patients in need thereof by administering a pharmaceutical composition comprising a compound of general formula I 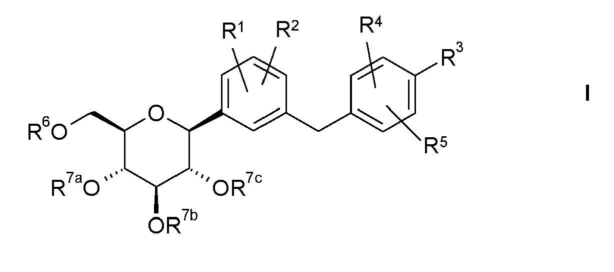 wherein the groups R¹ to R⁶ and R^{7a}, R^{7b}, R^{7c} are defined hereinafter, including the tautomers, the stereoisomers, the mixtures thereof and the salts thereof. In addition the present invention relates to the use of a compound of general formula I according to this invention for preparing a pharmaceutical composition for preventing and treating neurodegenerative disorders.

### BACKGROUND OF THE INVENTION

Glucopyranosyl-substituted benzene derivatives inhibit the sodium-dependent glucose cotransporters (SGLT), in particular SGLT2. Reuptake of filtered glucose across epithelial cells of the kidney proceeds via sodium-dependent glucose cotransporters (SGLTs) located in the brush-border membranes in the proximal tubuli along the sodium gradient ⁽¹⁾. There are at least 3 SGLT isoforms that differ in their expression pattern as well as in their physico-chemical properties ⁽²⁾. SGLT2 is exclusively expressed in the kidney ⁽³⁾. Under normoglycemia, glucose is completely reabsorbed by SGLTs in the kidney, whereas the reuptake capacity of the kidney is saturated at glucose concentrations higher than 10mM, resulting in glucosuria ("diabetes mellitus"). This threshold concentration can be decreased by SGLT2-inhibition. Renal filtration and reuptake of glucose contributes, among other mechanisms, to the steady state plasma glucose concentration and can therefore serve as an antidiabetic target. Therefore the glucopyranosyl-substituted benzene derivatives are proposed as inducers of urinary sugar excretion and as medicaments in the treatment of diabetes.
(1) Wright, E.M. (2001) Am. J. Renal Physiol. 280, F10-F18;
(2) Wright, E.M. et al. (2004) Pflugers Arch. 447(5):510-8;
(3) You, G. et al. (1995) J. Biol. Chem. 270 (49) 29365-29371;

Alzheimer's disease (AD) is a progressive neurodegenerative disorder characterized by multiple cognitive deficits including worsening of memory, judgement, and comprehension and deterioration in global functioning. As the disease progresses, motor, sensory, and linguistic abilities are also affected until there is global impairment of multiple cognitive functions. These cognitive losses occur gradually, but typically lead to severe impairment and eventual death in the range of four to twelve years. Current treatments are not efficacious in every patient.

Therefore there is an unmet medical need for drugs with a good efficacy with regard to the treatment, prevention or slowing, delaying or reversing progression of neurodegenerative disorders, such as dementia, in particular dementia of Alzheimer type, while at the same time showing an improved safety profile.

### AIM OF THE INVENTION

An aim of the present invention is to find a new method for treating of neurodegenerative disorders, in particular of a dementia.

Another aim of the present invention is to find a new method for preventing or slowing, delaying or reversing progression of neurodegenerative disorders, in particular of a dementia.

A further aim of the present invention is to find a new therapeutic use of a glucopyranosyl-substituted benzene derivative.

A further aim of the present invention is to provide new pharmaceutical compositions which are suitable for the treatment of neurodegenerative disorders, in particular dementia.

Other aims of the present invention will become apparent to the skilled man directly from the foregoing and following remarks.

### OBJECT OF THE INVENTION

In a first aspect the present invention relates to a method for treating of one or more neurodegenerative disorders in a patient in need thereof wherein said method comprises administering a glucopyranosyl-substituted benzene derivative of general formula (I) 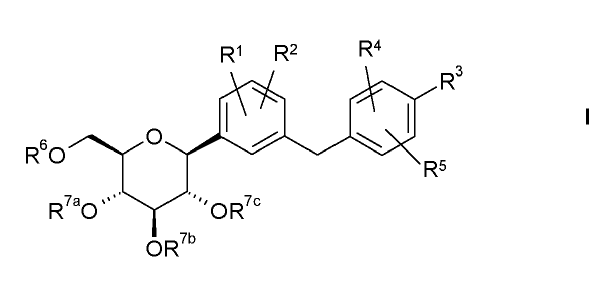 wherein
R¹ denotes hydrogen, fluorine, chlorine, bromine, iodine, cyano or nitro, or C₁₋₄-alkyl, a methyl group substituted by 1 to 3 fluorine atoms, an ethyl group substituted by 1 to 5 fluorine atoms, a C₁₋₄-alkyl group substituted by a hydroxy or C₁₋₃-alkoxy group, or
C₂₋₆-alken-1-yl, C₂₋₄-alkenyl-C₁₋₄-alkyl, C₂₋₆-alkyn-1-yl, C₂₋₄-alkynyl-C₁₋₄-alkyl, or C₂₋₄-alkenyl-C₁₋₄-alkoxy, C₂₋₄-alkynyl-C₁₋₄-alkoxy, or
C₃₋₇-cycloalkyl, C₃₋₇-cyclocloalkyl-C₁₋₄-alkyl, C₅₋₇-cycloalkenyl, C₅₋₇-cycloalkenyl-C₁₋₄-alkyl, or
hydroxy, C₁₋₄-alkoxy, a methoxy group substituted by 1 to 3 fluorine atoms, an ethoxy group substituted by 1 to 5 fluorine atoms, a C₂₋₄-alkoxy group substituted by a hydroxy or C₁₋₃-alkoxy group, or
C₃₋₇-cycloalkyloxy, C₅₋₇-cycloalkenyloxy, C₃₋₆-cydoalkyl-C₁₋₃-alkoxy or
C₁₋₄-alkylcarbonyl, aminocarbonyl, C₁₋₄-alkylaminocarbonyl, di-(C₁₋₃-alkyl)aminocarbonyl, pyrrolidin-1-ylcarbonyl, piperidin-1-ylcarbonyl, morpholin-4-ylcarbonyl, piperazin-1-ylcarbonyl, 4-(C₁₋₄-alkyl)piperazin-1-ylcarbonyl, C₁₋₄-alkoxycarbonyl, or amino, C₁₋₄-alkylamino, di-(C₁₋₃-alkyl)amino, pyrrolidin-1-yl, pyrrolidin-2-on-1-yl, piperidin-1-yl, piperidin-2-on-1-yl, morpholin-4-yl, morpholin-3-on-4-yl, piperazin-1-yl, 4-(C₁₋₃-alkyl)piperazin-1-yl, C₁₋₄-alkylcarbonylamino, or
C₁₋₄-alkylsulphinyl, C₁₋₄-alkylsulphonyl, C₃₋₇-cycloalkylsulphanyl, C₃₋₇-cycloalkylsulphinyl, C₃₋₇-cycloalkylsulphonyl, C₅₋₇-cycloalkenylsulphanyl, C₅₋₇-cycloalkenylsulphinyl, C₅₋₇-cycloalkenylsulphonyl, or
aryl, heteroaryl, aryloxy, heteroaryloxy, arylcarbonyl, heteroarylcarbonyl, arylaminocarbonyl, heteroarylaminocarbonyl, aryl-C₁₋₃-alkoxycarbonyl, heteroaryl-C₁₋₃-alkoxycarbonyl, arylcarbonylamino, heteroarylcarbonylamino, arylsulphanyl, arylsulphinyl, arylsulphonyl, heteroarylsulphanyl, heteroarylsulphinyl, heteroarylsul-phonyl,
while in the above-mentioned cycloalkyl and cycloalkenyl rings one or two methylene groups may be replaced independently of one another by O, S, CO, SO, SO₂ or NR^{N}, and
while the above-mentioned alkynyl and alkenyl groups may be mono- or polysubstituted by fluorine, and
the above-mentioned alkynyl and alkenyl groups may be mono- or disubstituted by identical or different groups L1, and
the above-mentioned cycloalkyl- and cycloalkenyl-rings independently of one another may be mono- or disubstituted by substituents selected from fluorine and C₁₋₃-alkyl, and
R² denotes fluorine, chlorine, bromine, iodine, hydroxy, amino, nitro, cyano, C₁₋₆-alkyl, C₂₋₆-alkenyl, C₂₋₆-alkynyl, C₃₋₇-cycloalkyl, C₃₋₇-cycloalkyl-C₁₋₃-alkyl, C₁₋₄-alkoxy, C₃₋₇-cycloalkyloxy, C₅₋₇-cydoalkenyloxy, C₁₋₄-alkylsulfanyl, while the alkyl or alkoxy group may be mono- or polysubstituted by fluorine; and
R³ denotes hydrogen, fluorine, chlorine, bromine, iodine, cyano, nitro, C₁₋₆-alkyl, a methyl or methoxy group substituted by 1 to 3 fluorine atoms, a C₂₋₄-alkyl or C₂₋₄-alkoxy group substituted by 1 to 5 fluorine atoms, a C₁₋₄-alkyl group substituted by a cyano group, a C₁₋₄-alkyl group substituted by a hydroxy or C₁₋₃-alkyloxy group, tri-(C₁₋₄-alkyl)silyl-C₁₋₆-alkyl,
C₂₋₆-alken-1-yl, C₂₋₄-alkenyl-C₁₋₄-alkyl,
C₂₋₆-alkyn-1-yl, C₂₋₄-alkynyl-C₁₋₄-alkyl,
C₂₋₄-alkenyl-C₁₋₄-alkoxy, C₂₋₄-alkynyl-C₁₋₄-alkoxy,
C₃₋₇-cycloalkyl, C₃₋₇-cycloalkyl-C₁₋₄-alkyl, C₅₋₇-cycloalkenyl, C₅₋₇-cycloalkenyl-C₁₋₄-alkyl, C₃₋₆-cycloalkylidenmethyl,
hydroxy, C₁₋₆-alkoxy, C₃₋₆-cycloalkyl-C₁₋₃-alkoxy, C₃₋₁₀-cycloalkyloxy, C₅₋₁₀-cycloalkenyloxy, or
C₃₋₇-cycloalkylethinyl, tetrahydrofuranylethinyl, tetrahydropyranylethinyl, C₃₋₇-cycloalkyloxy, tetrahydrofuranyloxy, tetrahydropyranyloxy or cycloalkanonyl, all of which may be substituted with one to four substituents L2, or
carboxy, C₁₋₃-alkoxycarbonyl, aminocarbonyl, (C₁₋₃-alkylamino)carbonyl, di-(C₁₋₃-alkyl)aminocarbonyl, pyrrolidin-1-ylcarbonyl, piperidin-1-ylcarbonyl, morpholin-4-ylcarbonyl, piperazin-1-yl-carbonyl, 4-(C₁₋₃-alkyl)-piperazin-1-ylcarbonyl, or
amino, C₁₋₃-alkylamino, di-(C₁₋₃-alkyl)amino, pyrrolidin-1-yl, pyrrolidin-2-on-1-yl, piperidin-1-yl, piperidin-2-on-1-yl, morpholin-4-yl, morpholin-3-on-4-yl, piperazin-1-yl, 4-(C₁₋₃-alkyl)piperazin-1-yl, (C₁₋₄-alkyl)carbonylamino, C₁₋₄-alkylsulphonylamino, or
C₁₋₄-alkylsulphanyl, C₁₋₄-alkylsulphinyl, C₁₋₄-alkylsulphonyl, C₃₋₁₀-cycloalkylsulphanyl, C₃₋₁₀-cycloalkylsulphinyl, C₃₋₁₀-cycloalkylsulphonyl, C₅₋₁₀-cycloalkenylsulphanyl, C₅₋₁₀-cycloalkenylsulphinyl, C₅₋₁₀-cycloalkenylsulphonyl, or
aryl, aryl-C₁₋₃-alkyl, arylcarbonylamino, heteroarylcarbonylamino, heteroaryl, heteroaryl-C₁₋₃-alkyl, aryloxy, aryl-C₁₋₃-alkyl-oxy, arylsulphanyl, arylsulphinyl, heteroarylsulphanyl or heteroarylsulphinyl, arylsulphonylamino, aryl-C₁₋₃-alkylsulphonylamino or arylsulphonyl, or
a arylethinyl-group or a 5- or 6-membered monocyclic heteroarylethinyl-group or a 5- or 6-membered monocyclic heteroaryloxy-group;
wherein a heteroaryl-group has 1 to 4 heteroatoms independently selected from the group consisting of N, O and S; and
wherein a heteroaryl-group may possess 1 or 2 carbonyl groups as part of the monocyclic aromatic ring-system; and
wherein an N-atom of a heteroaryl ring-system may be oxidized to form the corresponding N-oxide; and
wherein one or more methine groups in a aryl- and heteroaryl-group may be substituted independently of one another with a substituent L1; and
wherein one or more imino-groups in a heteroaryl-group may be substituted independently of one another with a substituent R^{N};
while the above-mentioned alkynyl and alkenyl groups may be mono- or polysubstituted by fluorine, and
the above-mentioned alkynyl and alkenyl groups may be mono- or disubstituted by identical or different groups L1; and
while the above-mentioned cycloalkyl and cycloalkenyl rings may be mono- or disubstituted independently of one another by substituents selected from fluorine and C₁₋₃-alkyl, and
in the above-mentioned cycloalkyl and cycloalkenyl rings one or two methylene groups may be replaced independently of one another by O, S, CO, SO, SO₂ or NR^{N},
R⁴ , R⁵ independently of each other denote hydrogen, fluorine, chlorine, bromine, iodine,
cyano, nitro, C₁₋₃-alkyl, C₁₋₃-alkoxy, methyl or methoxy substituted by 1 to 3 fluorine atoms, amino, C₁₋₃-alkyl-amino or di(C₁₋₃-alkyl)-amino; and
R^{N} denotes H, C₁₋₄-alkyl, C₁₋₄-alkylcarbonyl or C₁₋₄-alkylsulphonyl,
L1 independently of one another are selected from among hydroxy, cyano, nitro, C₃₋₇-cycloalkyl, C₁₋₄-alkylcarbonyl, aminocarbonyl, C₁₋₄-alkylaminocarbonyl, di-(C₁₋₃-alkyl)aminocarbonyl, C₁₋₄-alkoxycarbonyl and C₁₋₄-alkyloxy; and
L2 independently of one another are selected from among fluorine, chlorine, bromine,
iodine, C₁₋₃-alkyl, difluoromethyl, trifluoromethyl, C₁₋₃-alkoxy, difluoromethoxy, trifluoromethoxy and cyano; and
R⁶ R^{7a},
R^{7b}, R^{7c} independently of one another have a meaning selected from among hydrogen, (C₁₋₁₈-alkyl)carbonyl, (C₁₋₁₈-alkyl)oxycarbonyl, arylcarbonyl and aryl-(C₁₋₃-alkyl)-carbonyl,
while by the aryl groups mentioned in the definition of the above groups are meant phenyl or naphthyl groups which may be mono- or disubstituted independently of one another by identical or different groups L2; and
by the heteroaryl groups mentioned in the definition of the above groups are meant a pyrrolyl, furanyl, thienyl, pyridyl, indolyl, benzofuranyl, benzothiophenyl, quinolinyl, isoquinolinyl or tetrazolyl group,
or is meant a pyrrolyl, furanyl, thienyl or pyridyl group, wherein one or two methyne groups are replaced by nitrogen atoms,
or is meant an indolyl, benzofuranyl, benzothiophenyl, quinolinyl or isoquinolinyl group, wherein one to three methyne groups are replaced by nitrogen atoms,
while the above-mentioned heteroaryl groups independently of one another may be mono- or disubstituted by identical or different groups L2;
while, unless otherwise stated, the above-mentioned alkyl groups may be straight-chain or branched,
a tautomer thereof, a stereoisomer thereof, a mixture of compounds of the general formula (I) or a salt thereof,
to the patient in need thereof.

In a further aspect the present invention relates to a method for preventing or slowing, delaying or reversing progression of one or more neurodegenerative disorders in a patient in need thereof wherein said method comprises administering a glucopyranosyl-substituted benzene derivative of general formula (I), a tautomer, stereoisomer, mixture or salt thereof, as defined hereinbefore and hereinafter to the patient in need thereof.

Another aspect of the present invention relates to the use of a glucopyranosyl-substituted benzene derivative of general formula (I), a tautomer, stereoisomer, mixture or salt thereof, as defined hereinbefore and hereinafter for the manufacture of a medicament for the treatment of one or more neurodegenerative disorders.

Another aspect of the present invention relates to the use of a glucopyranosyl-substituted benzene derivative of general formula (I), a tautomer, stereoisomer, mixture or salt thereof, as hereinbefore and hereinafter for the manufacture of a medicament for preventing or slowing, delaying or reversing progression of one or more neurodegenerative disorders.

Another aspect of the present invention relates to a pharmaceutical composition for the treatment of one or more neurodegenerative disorders comprising a glucopyranosyl-substituted benzene derivative of general formula (I), a tautomer, stereoisomer, mixture or salt thereof, as defined hereinbefore and hereinafter.

Another aspect of the present invention relates to a pharmaceutical composition for preventing or slowing, delaying or reversing progression of one or more neurodegenerative disorders comprising a glucopyranosyl-substituted benzene derivative of general formula (I), a tautomer, stereoisomer, mixture or salt thereof, as defined hereinbefore and hereinafter.

### DETAILED DESCRIPTION OF THE INVENTION

Unless otherwise stated the groups, residues and substituents, particularly R¹ to R⁶ and R^{7a}, R^{7b} , R^{7c} , are defined as above and hereinafter.

If residues, substituents or groups occur several times in a compound, they may have the same or different meanings.

The group R¹ preferably denotes hydrogen, fluorine, chlorine, bromine, iodine, amino, nitro or cyano, hydroxy, C₁₋₄-alkyl, methyl substituted by 1 to 3 fluorine atoms, ethyl substituted by 1 to 5 fluorine atoms, C₁₋₄-alkyl substituted by a hydroxy or C₁₋₃-alkoxy group, C₂₋₆-alkenyl, C₂₋₆-alkynyl, C₁₋₄-alkoxy, methoxy substituted by 1 to 3 fluorine atoms, ethoxy substituted by 1 to 5 fluorine atoms, C₂₋₄-alkoxy substituted by a hydroxy or C₁₋₃-alkoxy group, C₂₋₄-alkenyl-C₁₋₄-alkoxy, C₂₋₄-alkynyl-C₁₋₄-alkoxy, C₃₋₆-cycloalkyl, C₃₋₆-cycloalkyl-C₁₋₃-alkyl, C₃₋₇-cycloalkyloxy, C₃₋₆-cycloalkyl-C₁₋₃-alkoxy or C₅₋₇-cycloalkenyloxy, while in the C₅₋₆-cycloalkyl groups a methylene group may be replaced by O.

Even more preferably the group R¹ denotes hydrogen, fluorine, chlorine, bromine, cyano, methyl, ethyl, isopropyl, difluoromethyl, trifluoromethyl, ethynyl, prop-1-yn-1-yl, but-1-yn-1-yl, hydroxy, methoxy, ethoxy, difluoromethoxy, cyclopropyloxy, cyclobutyloxy, cyclopentyloxy, cyclohexyloxy, tetrahydrofuran-3-yloxy or tetrahydropyran-4-yl-oxy.

Most preferred meanings of the group R¹ are methyl, chlorine, cyano and cyclopropyl.

The group R² preferably denotes hydrogen, fluorine, chlorine, bromine, cyano, nitro, methyl, methyl substituted by 1 to 3 fluorine atoms, hydroxy, methoxy, ethoxy, trifluoromethoxy, isopropoxy, cyclopropyl, cyclobutyl, cyclopentyl, cyclopropyloxy, cyclobutyloxy, cyclopentyloxy.

According to a first preferred embodiment the group R¹ denotes cyano and R² denotes hydrogen.

According to a second preferred embodiment the group R¹ denotes cyano and R² is defined as hereinbefore, but R² does not denote hydrogen.

The group R³ preferably denotes hydrogen, fluorine, chlorine, methyl, ethyl, isopropyl, tert.-butyl, ethynyl, 1-propynyl, trimethylsilylethyl, difluoromethyl, trifluoromethyl, cyclopropyl, cyclobutyl, cyclopentyl, methoxy, ethoxy, isopropoxy, cyclopentyloxy, difluoromethoxy, trifluoromethoxy, pentafluorethoxy, tetrahydrofuran-3-yloxy, tetrahydrofuran-2-on-3-yloxy, methylsulphanyl, ethylsulphanyl, isopropylsulphanyl, cyclopropylidenemethyl, phenyl, fluorophenyl, pyridinyl, pyrimidinyl, pyridazinyl, pyrazinyl, imidazolyl, pyrazolyl, triazolyl, tetrazolyl, oxazolyl, oxadiazolyl, thiazolyl, thiadiazolyl, trimethylsilylethyl, ethynyl, 1-propyn-1-yl, 1-butyn-1-yl, tert.-butylethynyl, 2-hydroxyprop-2-ylethynyl, 2-methoxyprop-2-ylethynyl, 3-hydroxy-1-propyn-1-yl, 3-methoxy-1-propyn-1-yl, ethenyl, 1-propenyl, 1-butenyl, tert.-butylethenyl, cyclopropyloxy, cyclobutyloxy, cyclopentyloxy, cyclohexyloxy, tetrahydrofuranyloxy, tetrahydrothiophenyloxy, 1,1-dioxotetrahydrothiophenyloxy, tetrahydropyranyloxy, tetrahydrothiopyranyloxy, 1,1-dioxotetrahydrothiopyranyloxy, tetrahy-drofuranonyloxy, piperidinyloxy, piperidinonyloxy, pyrrolidin-3-yloxy, pyrrolidinon-3-yloxy, tetrahydrofuranyl-sulphanyl, cyclopropylsulphanyl, cyclobutylsulphanyl, cyclopentyl-sulphanyl and cyclohexylsulphanyl, while the -NH group in a piperidinyl, piperidinonyl, pyrrolidinyl or pyrrolidinonyl ring may be substituted by R^{N}, particularly C₁₋₃-alkyl or acetyl; or
1-hydroxy-cyclopropylethinyl, 1-hydroxy-cyclobutylethinyl, 1-hydroxy-cyclopentylethinyl, 1-hydroxy-cyclohexylethinyl, tetrahydrofuran-2-ylethinyl, tetrahydrofuran-3-ylethinyl, tetrahydropyran-4-ylethinyl, 4-hydroxy-tetrahydropyran-4-ylethinyl, 1-methoxy-cyclopropylethinyl, 1-methoxy-cyclobutylethinyl, 1-methoxy-cyclopentylethinyl, 1-methoxy-cyclohexylethinyl, 4-methoxy-tetrahydropyran-4-ylethinyl, 1-hydroxymethyl-cyclopropylethinyl, 1-hydroxymethyl-cyclobutylethinyl, 1-hydroxymethyl-cyclopentylethinyl, 1-hydroxymethyl-cyclohexylethinyl, 4-hydroxymethyl-tetrahydropyran-4-ylethinyl, all of which may be substituted with an additional substituent L2; or 2-hydroxy-cyclopropyloxy, 2-hydroxy-cyclobutyloxy, 3-hydroxy-cyclobutyloxy, 2-hydroxy-cyclopentyloxy, 3-hydroxy-cyclopentyloxy, 2-hydroxy-cyclohexyloxy, 3-hydroxy-cyclohexyloxy, 4-hydroxy-cyclohexyloxy, 2-methoxy-cyclopropyloxy, 2-methoxy-cyclobutyloxy, 3-methoxy-cyclobutyloxy, 2-methoxy-cyclopentyloxy, 3-methoxy-cyclopentyloxy, 2-methoxy-cyclohexyloxy, 3-methoxy-cyclohexyloxy, 4-methoxy-cyclohexyloxy, 1-hydroxymethyl-cyclopentyloxy, 1-hydroxymethyl-cyclohexyloxy, 1-methoxymethyl-cyclopentyloxy, 1-methoxymethyl-cyclohexyloxy, 4-hydroxy-tetrahydrofuran-3-yloxy, 4-methoxy-tetrahydrofuran-3-yloxy, 3-hydroxy-tetrahydropyran-4-yloxy and 4-hydroxy-tetrahydropyran-3-yloxy, all of which may be substituted with an additional substituent L2; or
2-oxo-cyclopentyl and 2-oxo-cyclohexyl, which may be substituted with an additional substituent L2; or
phenylethinyl, pyridylethinyl, pyridazinylethinyl, pyrazinylethinyl, pyrimidinylethinyl, thienylethinyl, thiazolylethinyl, oxazolylethinyl, isoxazolylethinyl, [1,2,4]oxadiazolylethinyl, [1H-[1,2,4]triazolyl]ethinyl, [2H-tetrazolyl]ethinyl, [1,2-dihydro-2-oxo-pyridinyl]ethinyl or [1,2,3,4-tetrahydro-2,4-dioxo-pyrimidinyl]ethinyl, wherein one or more methine-groups in said phenyl or said heteroaryl-groups may be substituted independently of one another with a substituent L1; and
pyridyloxy, pyridazinyloxy, pyrazinyloxy, pyrimidinyloxy, pyrazolyloxy, imidazolyloxy, triazinyloxy, thienyloxy, thiazolyloxy, oxazolyloxy, isoxazolyloxy, [1,2,4]oxadiazolyloxy, [1 H-[1,2,4]triazolyl]oxy, or [2H-tetrazolyl]oxy,
wherein one or more methine-groups in said heteroaryl-groups may be substituted independently of one another with a substituent L1; and
wherein one or more imino-groups in said heteroaryl-groups may be substituted independently of one another with a substituent R^{N}.

Even more preferably the group R³ denotes hydrogen, fluorine, chlorine, bromine, iodine, cyano, methyl, ethyl, propyl, isopropyl, butyl, sec-butyl, iso-butyl, tert-butyl, 3-methyl-but-1-yl, cyclopropyl, cyclobutyl, cyclopentyl, cyclohexyl, cyclopropylidenemethyl, difluoromethyl, trifluoromethyl, pentafluoroethyl, 2-hydroxyl-ethyl, hydroxymethyl, 3-hydroxypropyl, 2-hydroxy-2-methyl-prop-1-yl, 3-hydroxy-3-methyl-but-1-yl, 1-hydroxy-1-methylethyl, 2,2,2-trifluoro-1-hydroxy-1-methyl-ethyl, 2,2,2-trifluoro-1-hydroxy-1-trifluoromethylethyl, 2-methoxy-ethyl, 2-ethoxy-ethyl, hydroxy, methyloxy, ethyloxy, isopropyloxy, difluoromethyloxy, trifluoromethyloxy, pentafluorethoxy, cyclobutyloxy, cyclopentyloxy, cyclohexyloxy, (*S*)-tetrahydrofuran-3-yloxy, (*R*)-tetrahydrofuran-3-yloxy, tetrahydropyran-4-yloxy, tetrahydrofuran-2-on-3-yloxy, 1-acetyl-piperidin-4-yloxy, 2-methyloxy-ethyloxy, methylsulfanyl, ethylsulphanyl, isopropylsulphanyl, methylsulfinyl, methlysulfonyl, ethylsulfinyl, ethylsulfonyl, trimethylsilyl, trimethylsilylethyl, ethynyl, 2-hydroxyprop-2-ylethynyl, 2-methoxyprop-2-ylethynyl, 3-hydroxy-1-propyn-1-yl, 3-methoxy-1-propyn-1-yl, cyclopropyloxy, cyclobutyloxy, cyclopentyloxy, cyclohexyloxy, tetrahydrofuran-3-yloxy, tetrahydropyran-4-yloxy, piperidin-4-yloxy, N-methylpiperidin-4-yloxy or N-acetylpiperidin-4-yloxy.

The groups R⁴, R⁵ preferably denote independently of each other hydrogen, fluorine, hydroxy, methoxy, ethoxy or methyl, particularly hydrogen or methyl.

According to a preferred embodiment R⁴ and R⁵ denote H.

According to another preferred embodiment R⁴ denotes H and R⁵ denotes F.

According to another preferred embodiment R⁴ denotes F and R⁵ denotes H.

According to another preferred embodiment R⁴ and R⁵ denote F.

The group L1 preferably denotes fluorine, hydroxy, hydroxy-C₁₋₄-alkyl, C₁₋₄-alkoxy, C₁₋₄-alkoxy-C₁₋₄-alkyl, C₁₋₄-alkyl, trifluoromethyl, C₁₋₄-alkyl-carbonylamino, hydroxycarbonyl or C₁₋₄-alkoxycarbonyl; particularly fluorine, hydroxy, hydroxymethyl, methoxy or methyl.

The group L2 preferably denotes fluorine, hydroxy, hydroxy-C₁₋₄-alkyl, C₁₋₄-alkoxy, C₁₋₄-alkoxy-C₁₋₄-alkyl, C₁₋₄-alkyl, trifluoromethyl, C₁₋₄-alkyl-carbonylamino, hydroxycarbonyl or C₁₋₄-alkoxycarbonyl; particularly hydroxy, hydroxymethyl, methoxy or methyl.

The group R^{N} preferably denotes C₁₋₃-alkyl or acetyl, in particular methyl.

The group R⁶ preferably denotes according to the invention hydrogen, (C₁₋₈-alkyl)oxycarbonyl, C₁₋₈-alkylcarbonyl or benzoyl, particularly hydrogen or (C₁₋₆-alkyl)oxycarbonyl or C₁₋₆-alkylcarbonyl, particularly preferably hydrogen, methylcarbonyl, methoxycarbonyl or ethoxycarbonyl, most particularly preferably hydrogen.

The substituents R^{7a}, R^{7b}, R^{7c} preferably represent independently of one another hydrogen, (C₁₋₈-alkyl)oxycarbonyl, (C₁₋₁₈-alkyl)carbonyl or benzoyl, particularly hydrogen, (C₁₋₆-alkyl)oxycarbonyl or (C₁₋₈-alkyl)carbonyl, particularly preferably hydrogen, methoxycarbonyl, ethoxycarbonyl, methylcarbonyl or ethylcarbonyl. Most particularly preferably R^{7a}, R^{7b} and R^{7c} represent hydrogen.

In the methods, uses and pharmaceutical compositions according to this invention compounds of the formula (la) and (Ib) are preferred 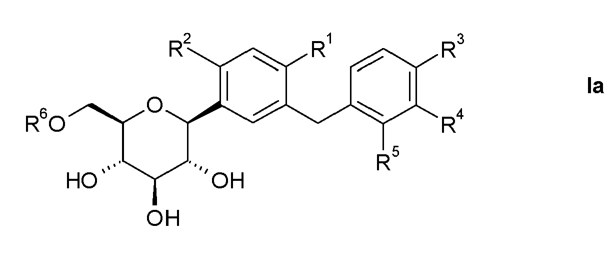 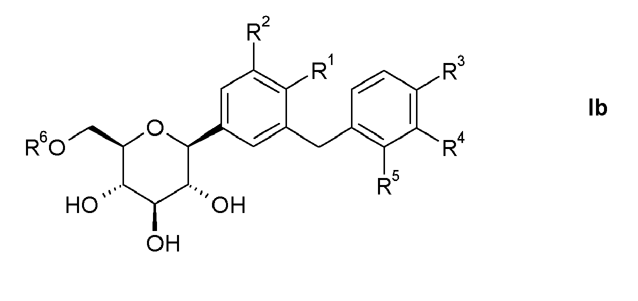 wherein R¹ to R⁶ are defined as hereinbefore.

In the methods, uses and pharmaceutical compositions according to this invention the following compounds (1) to (382) are particularly preferred:
Preferred compounds according to this invention are selected from the following table:

| Ex. | Struktur | Ex. | Struktur |
|---|---|---|---|
| 1 | 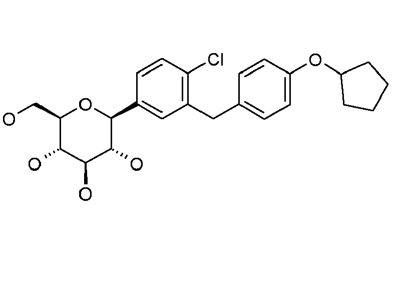 | 2 | 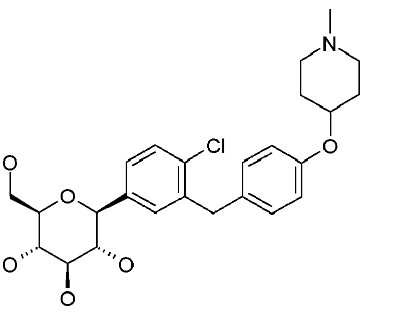 |
| 3 | 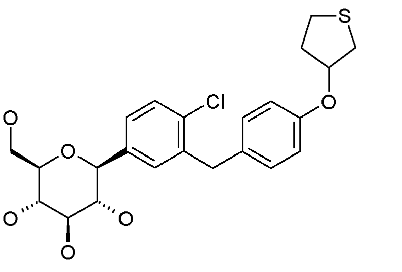 | 4 | 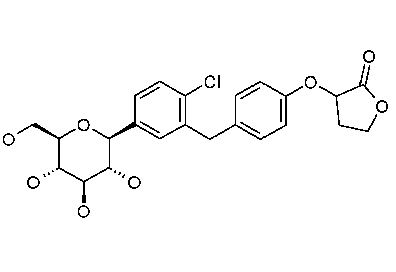 |
| 5 | 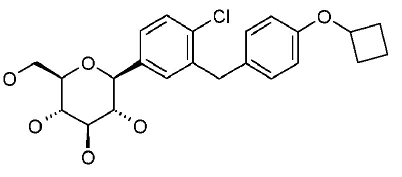 | 6 | 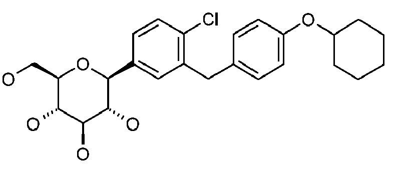 |
| 7 | 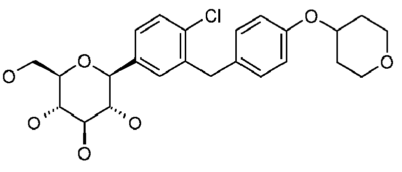 | 8 | 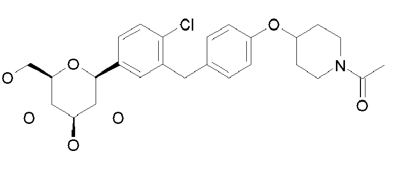 |
| 9 | 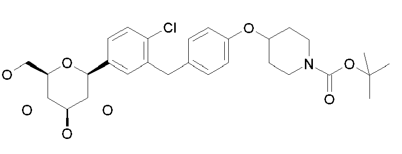 | 10 | 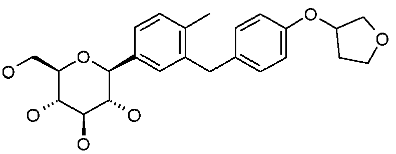 |
| 11 | 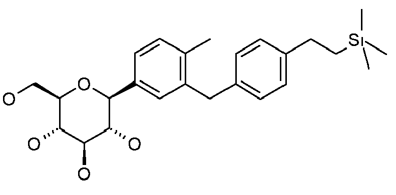 | 12 | 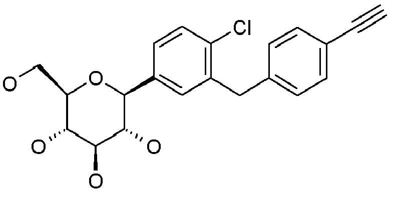 |
| 13 | 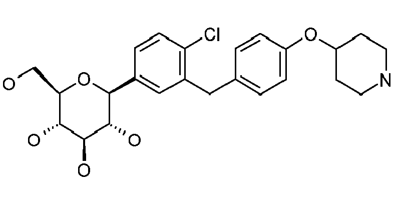 | 14 | 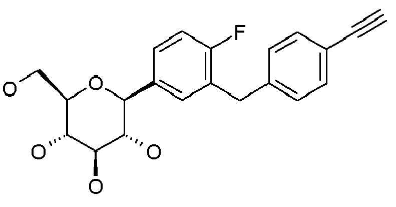 |
| 15 | 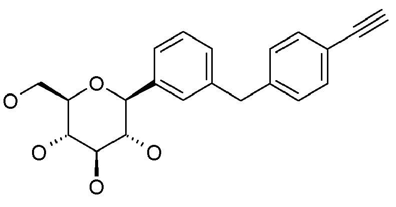 | 16 | 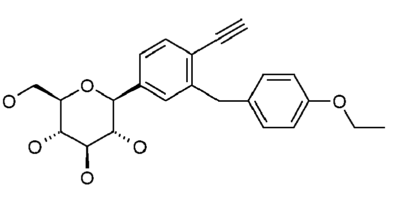 |
| 17 | 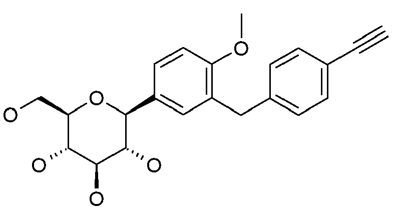 | 18 | 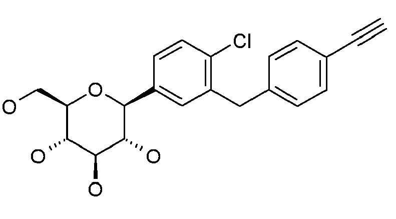 |
| 19 | 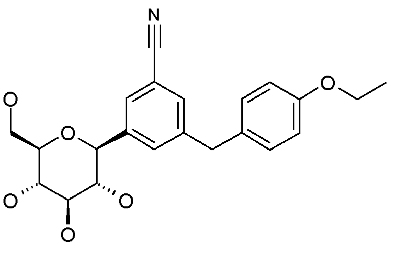 | 20 | 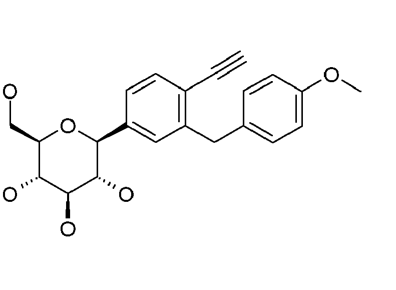 |
| 21 | 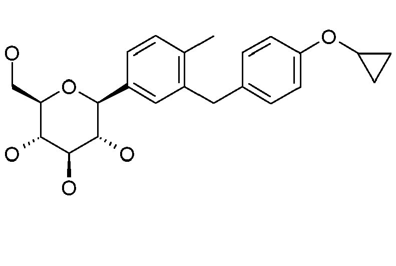 | 22 | 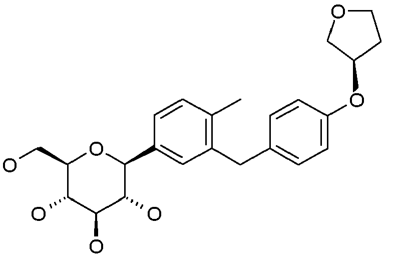 |
| 23 | 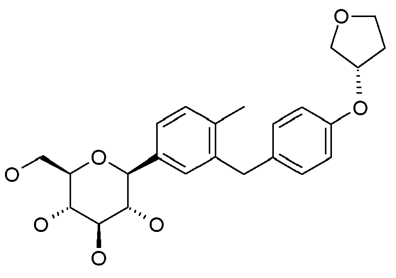 | 24 | 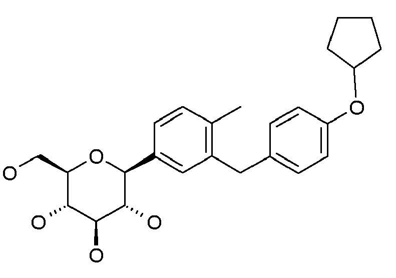 |
| 25 | 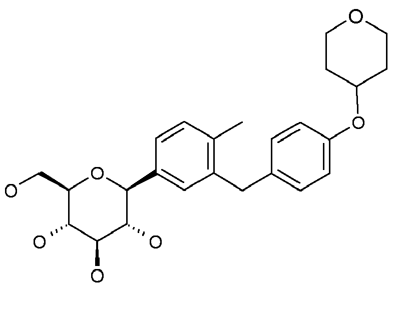 | 26 | 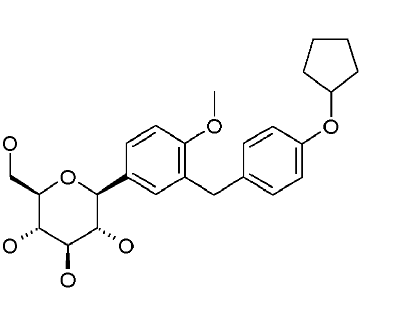 |
| 27 | 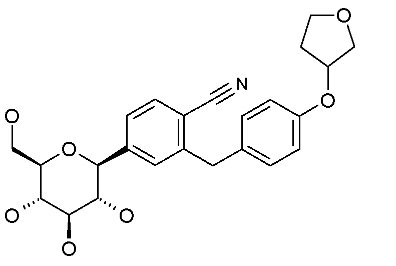 | 28 | 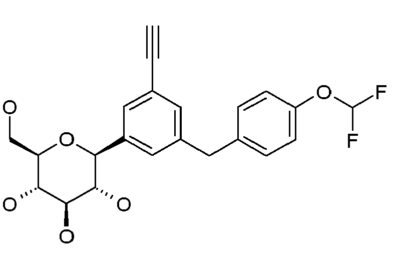 |
| 29 | 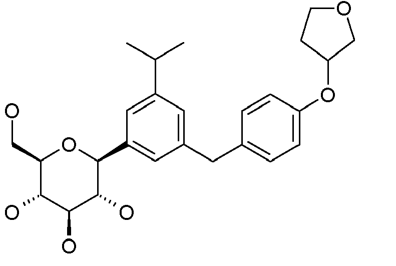 | 30 | 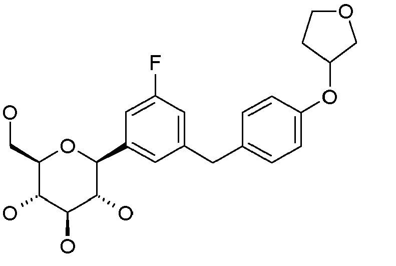 |
| 31 | 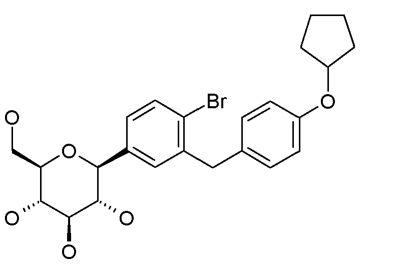 | 32 | 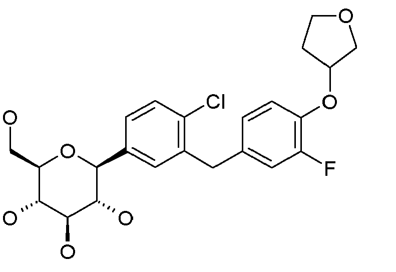 |
| 33 | 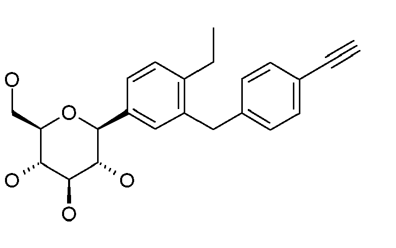 | 34 | 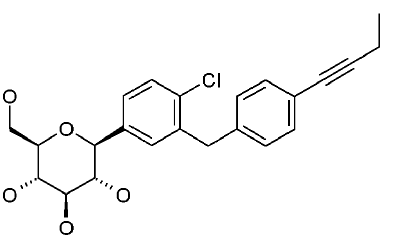 |
| 35 | 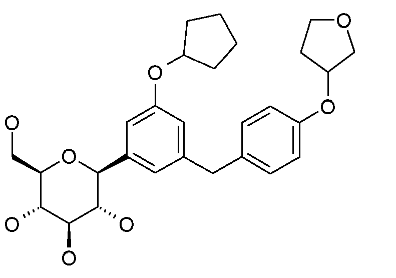 | 36 | 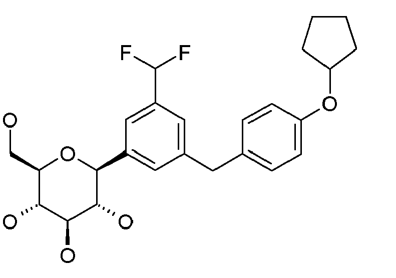 |
| 37 | 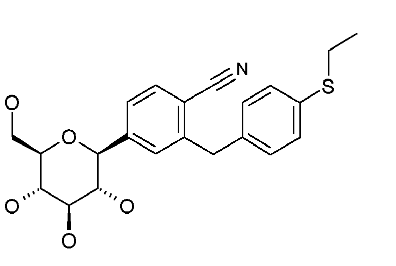 | 38 | 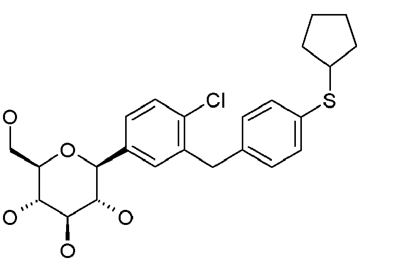 |
| 39 | 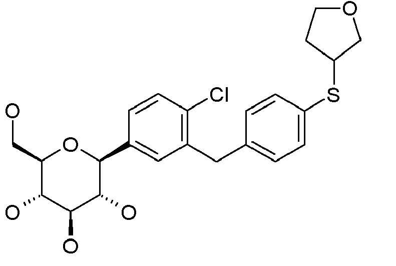 | 40 | 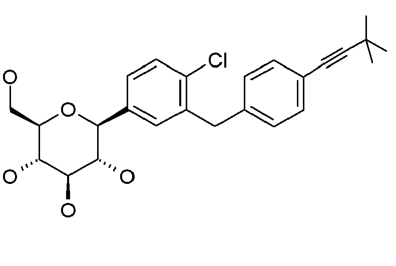 |
| 41 | 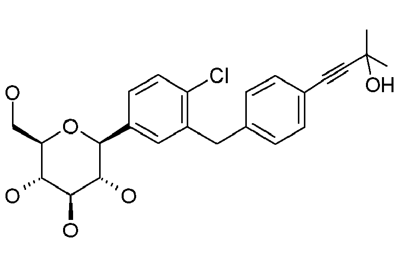 | 42 | 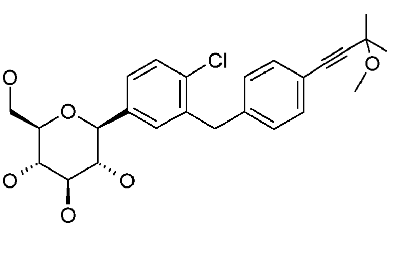 |
| 43 | 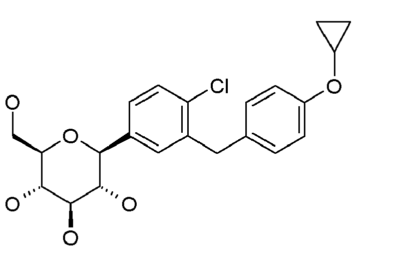 | 44 | 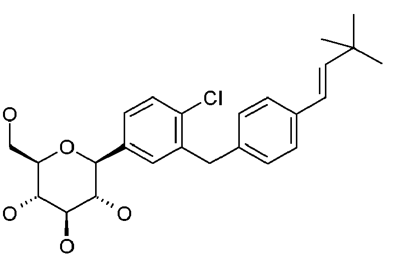 |
| 45 | 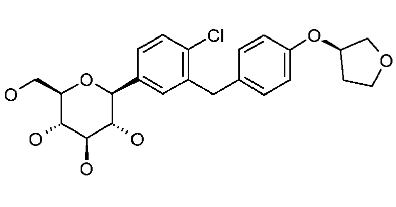 | 46 | 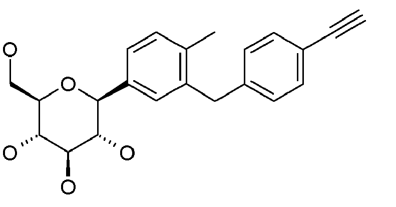 |
| 47 | 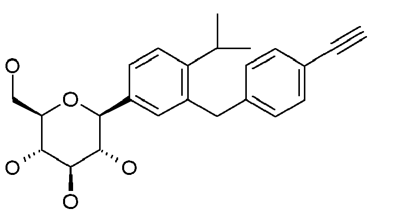 | 48 | 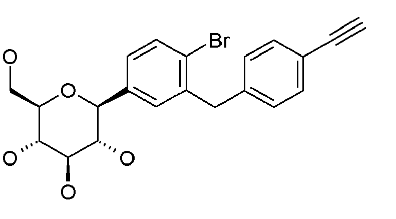 |
| 49 | 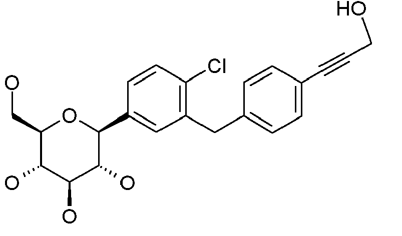 | 50 | 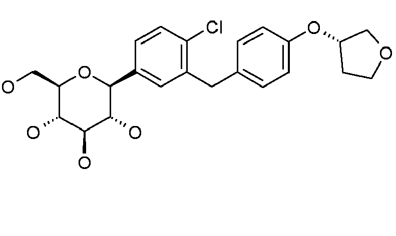 |
| 51 | 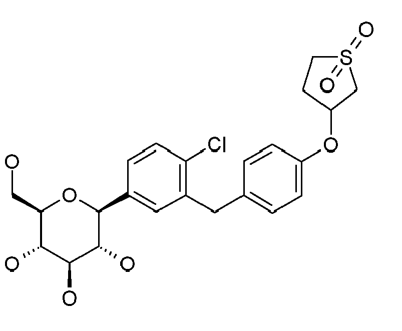 | 52 | 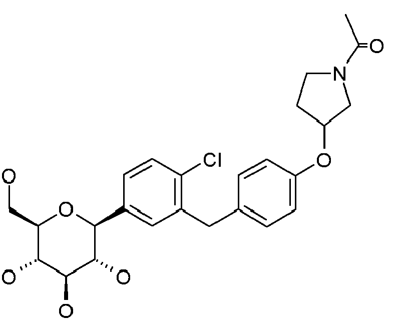 |
| 53 | 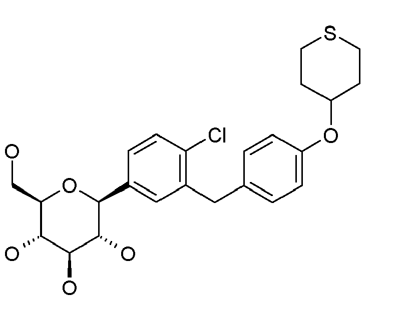 | 54 | 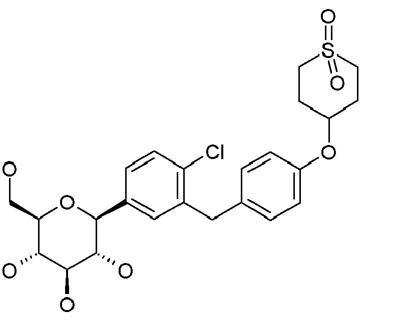 |
| 55 | 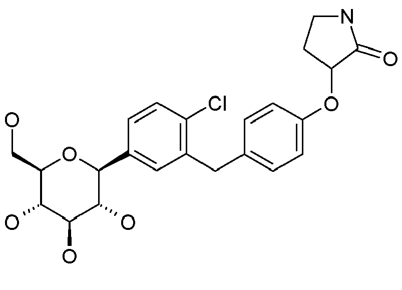 | 56 | 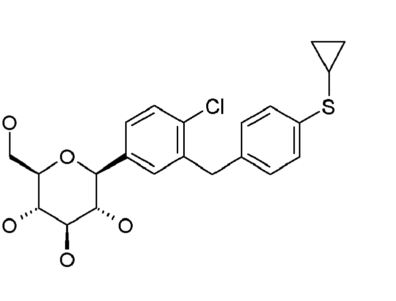 |
| 57 | 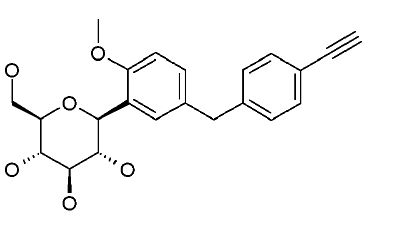 | 58 | 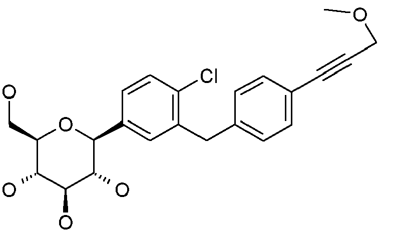 |
| 59 | 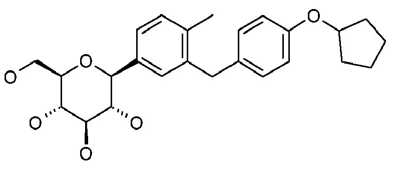 | 60 | 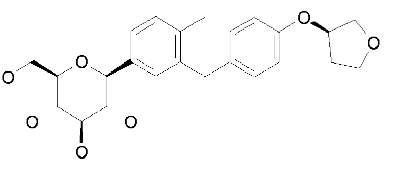 |
| 61 | 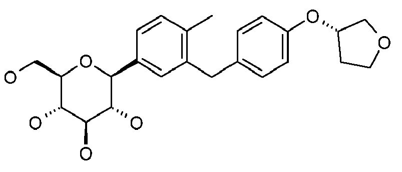 | 62 | 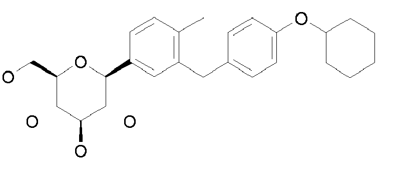 |
| 63 | 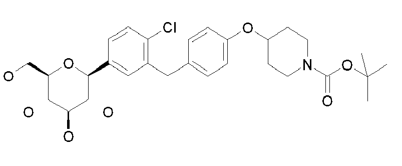 | 64 | 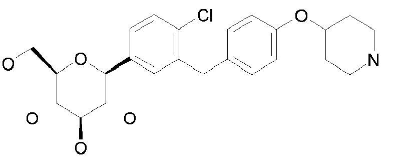 |
| 65 | 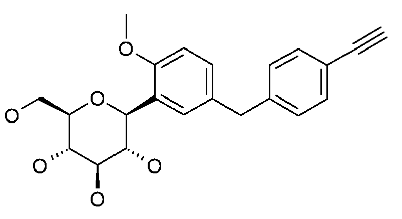 | 66 | 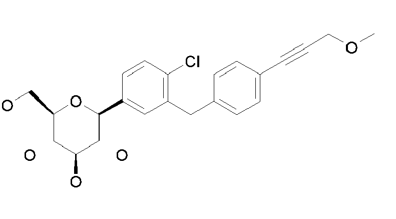 |
| 67 | 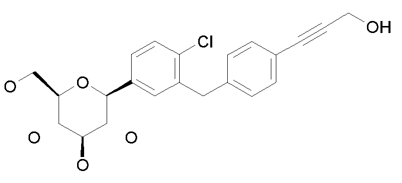 | 68 | 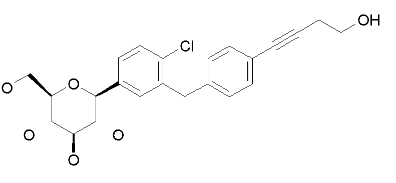 |
| 69 | 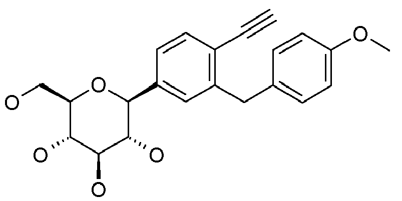 | 70 | 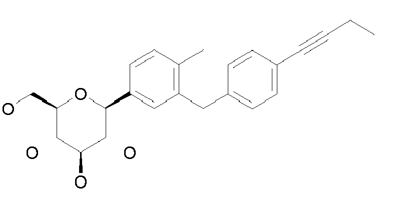 |
| 71 | 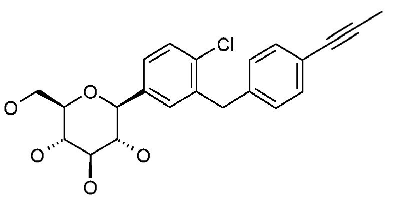 | 72 | 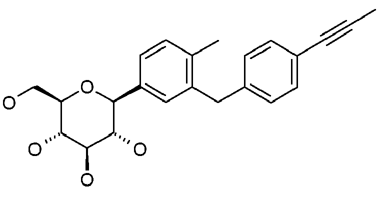 |
| 73 | 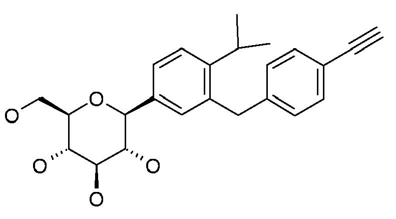 | 74 | 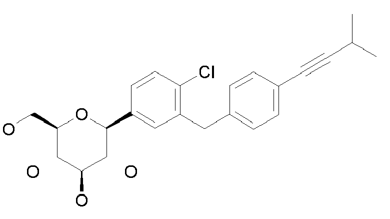 |
| 75 | 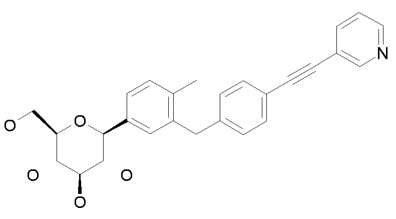 | 76 | 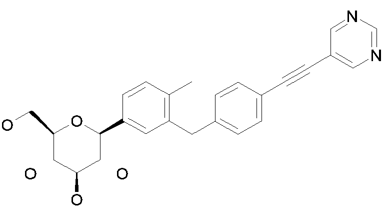 |
| 77 | 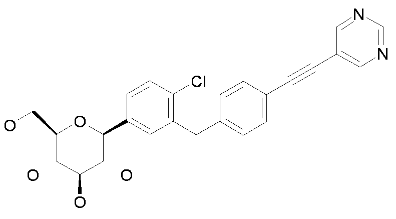 | 78 | 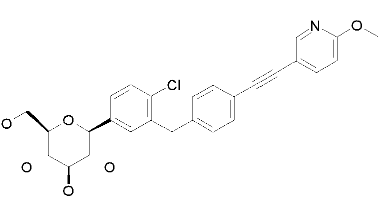 |
| 79 | 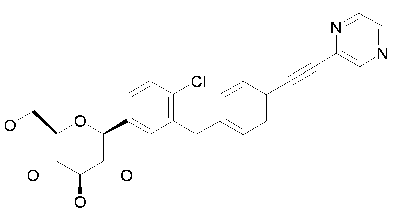 | 80 | 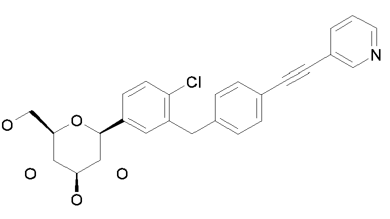 |
| 81 | 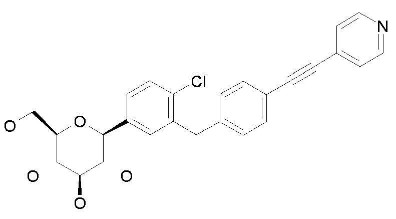 | 82 | 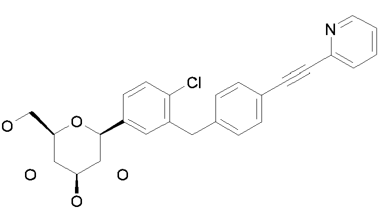 |
| 83 | 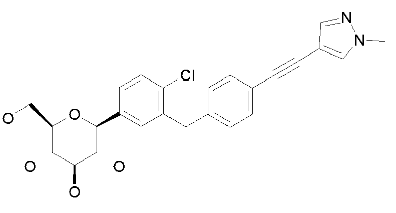 | 84 | 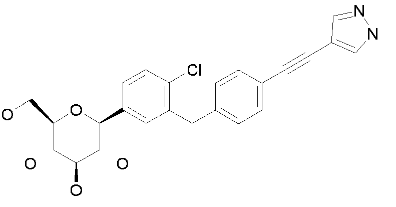 |
| 85 | 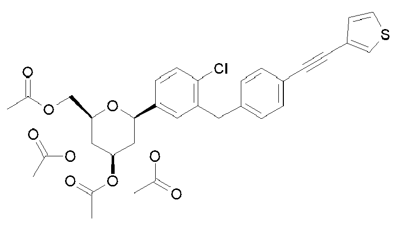 | 86 | 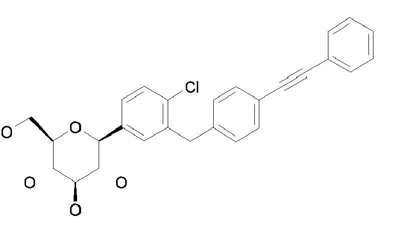 |
| 87 | 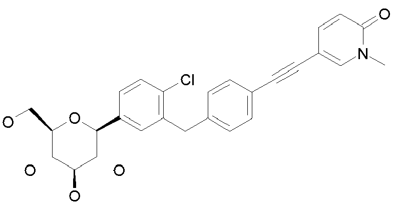 | 88 | 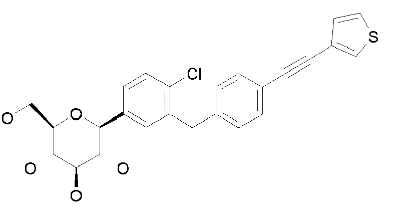 |
| 89 | 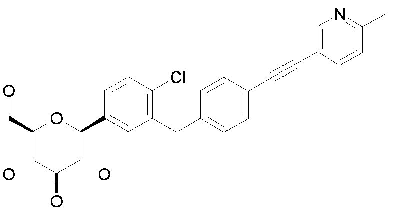 | 90 | 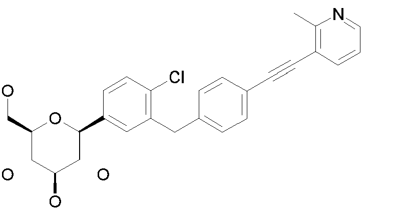 |
| 91 | 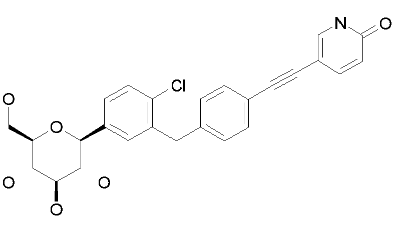 | 92 | 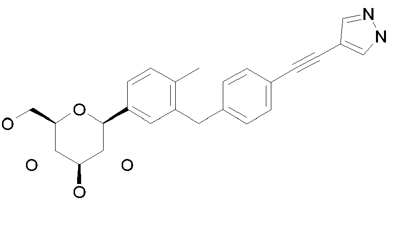 |
| 93 | 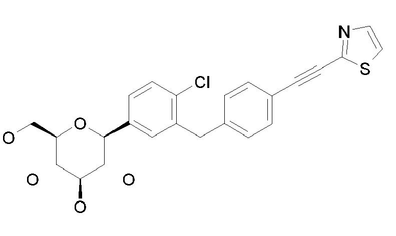 | 94 | 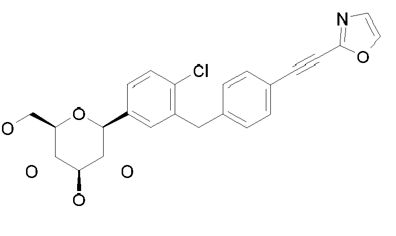 |
| 95 | 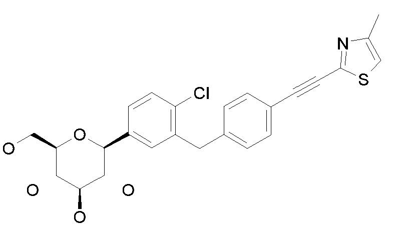 | 96 | 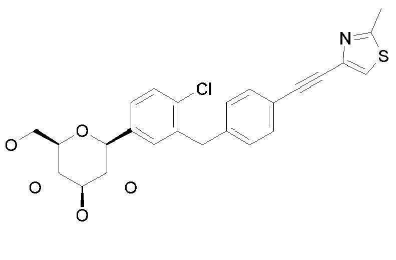 |
| 97 | 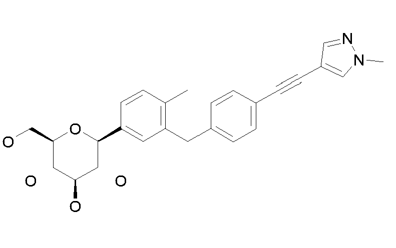 | 98 | 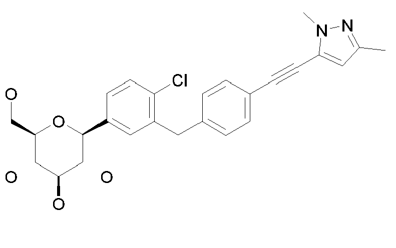 |
| 99 | 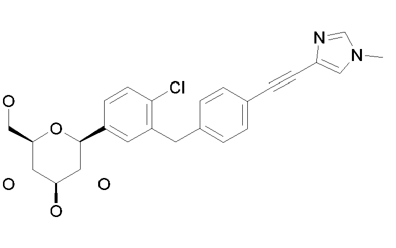 | 100 | 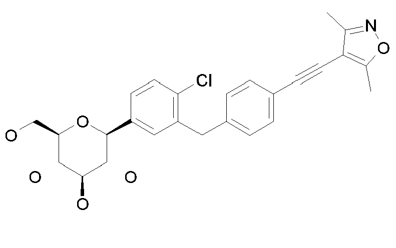 |
| 101 | 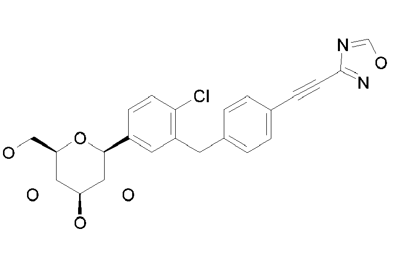 | 102 | 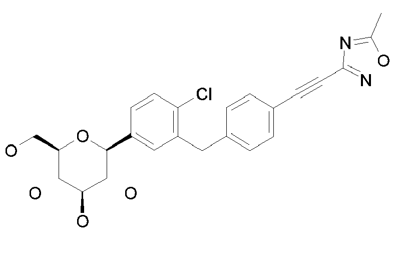 |
| 103 | 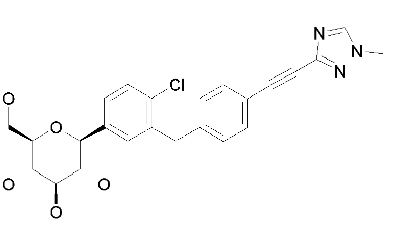 | 104 | 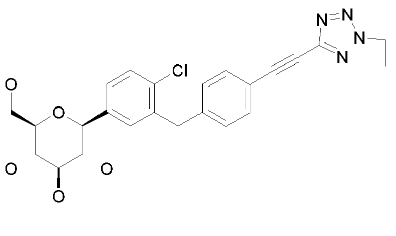 |
| 105 | 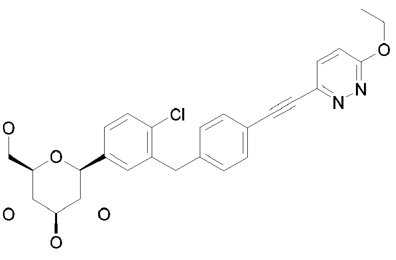 | 106 | 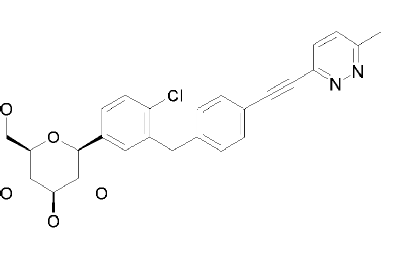 |
| 107 | 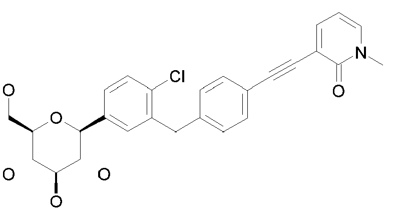 | 108 | 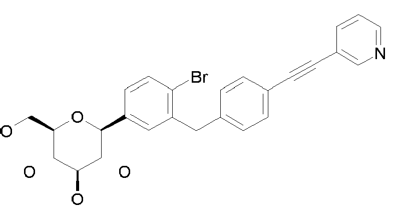 |
| 109 | 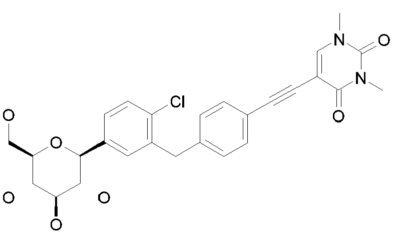 | 110 | 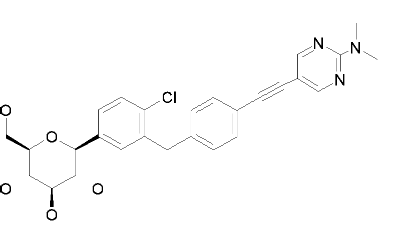 |
| 111 | 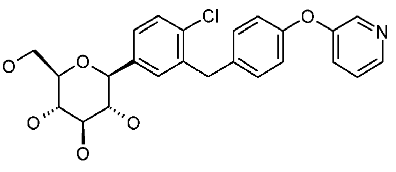 | 112 | 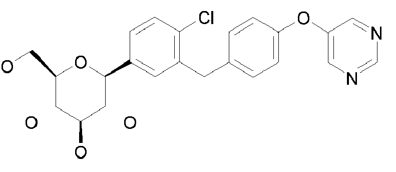 |
| 113 | 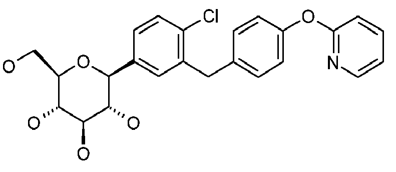 | 114 | 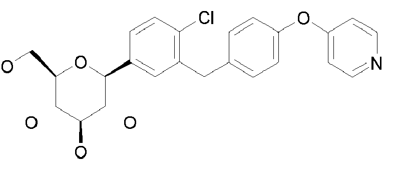 |
| 115 | 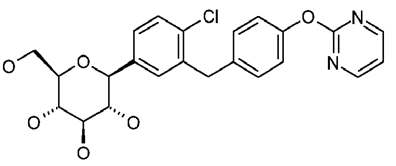 | 116 | 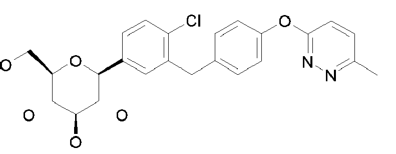 |
| 117 | 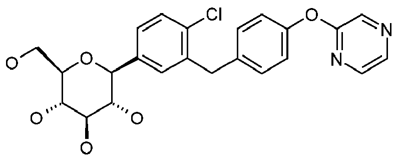 | 118 | 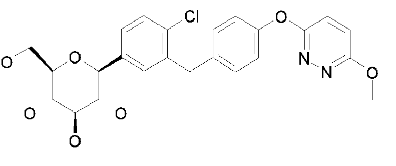 |
| 119 | 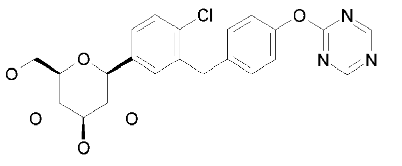 | 120 | 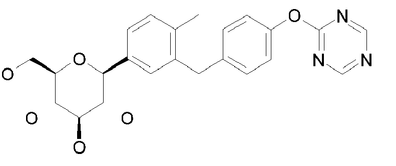 |
| 121 | 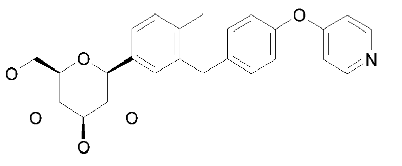 | 122 | 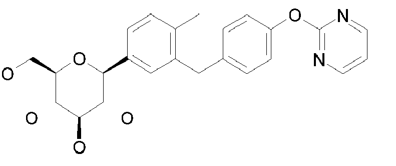 |
| 123 | 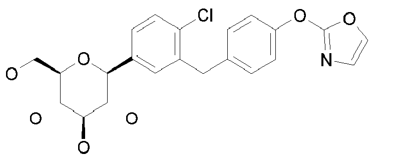 | 124 | 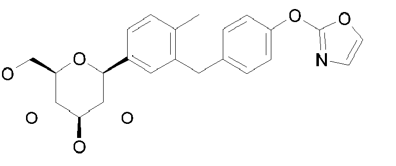 |
| 125 | 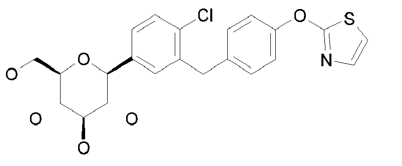 | 126 | 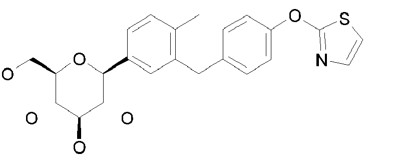 |
| 127 | 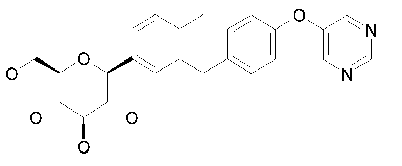 | 128 | 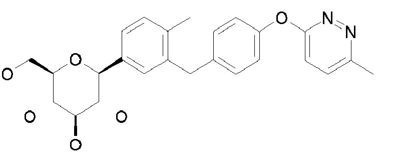 |
| 129 | 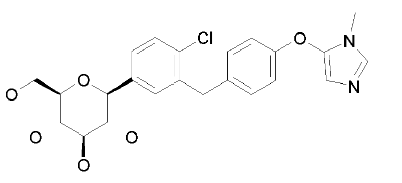 | 130 | 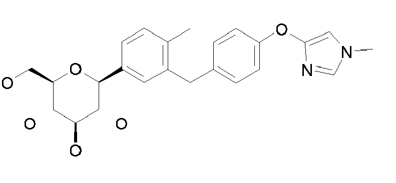 |
| 131 | 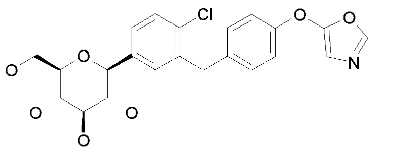 | 132 | 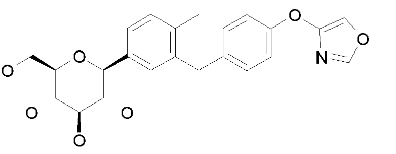 |
| 133 | 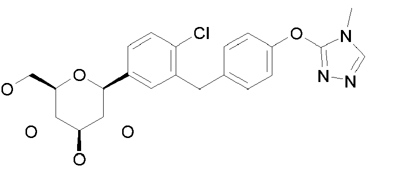 | 134 | 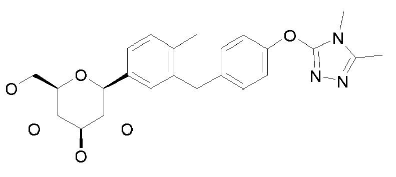 |
| 135 | 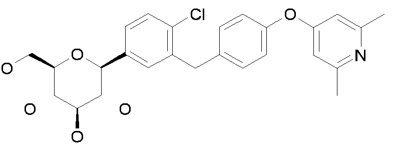 | 136 | 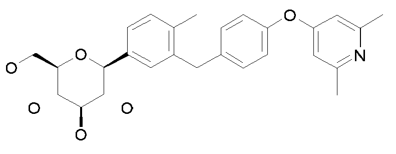 |
| 137 | 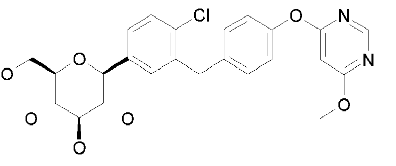 | 138 | 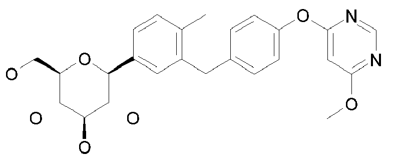 |
| 139 | 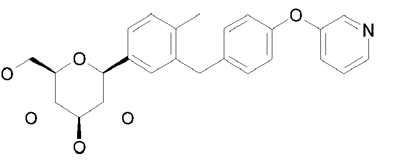 | 140 | 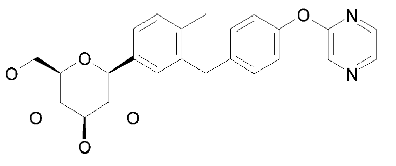 |
| 141 | 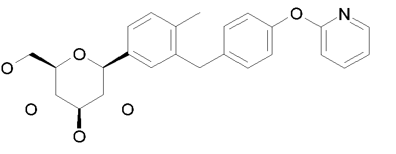 | 142 | 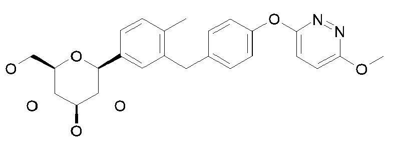 |
| 143 | 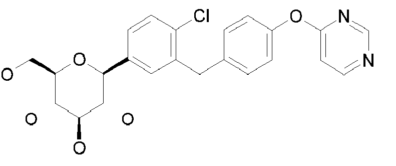 | 144 | 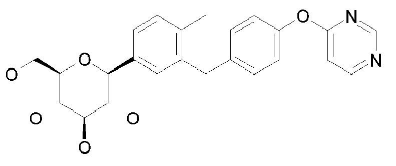 |
| 145 | 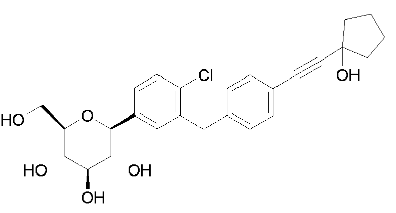 146 | 146 | 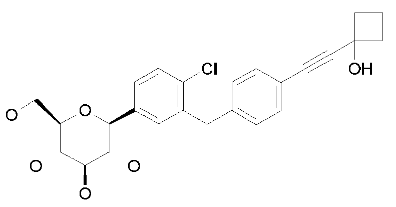 |
| 147 | 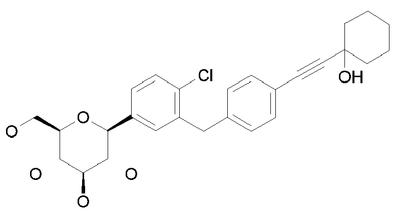 | 148 | 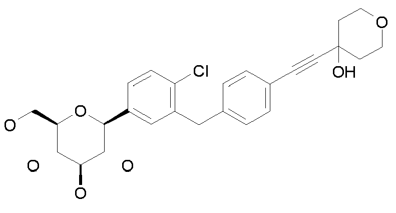 |
| 149 | 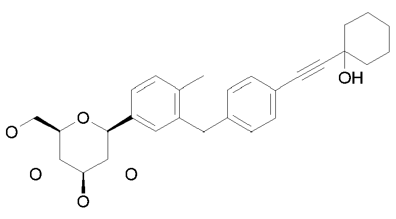 | 150 | 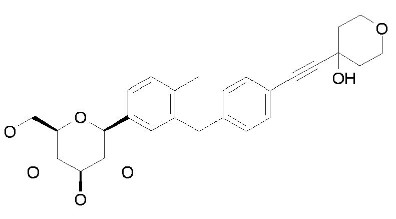 |
| 151 | 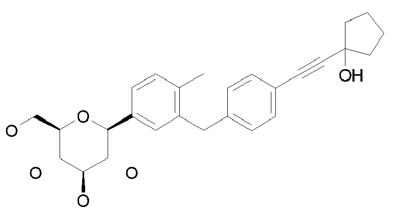 | 152 | 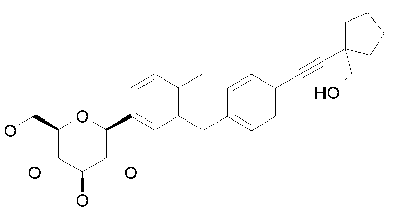 |
| 153 | 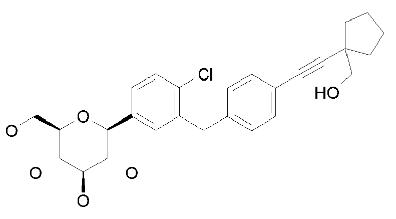 | 154 | 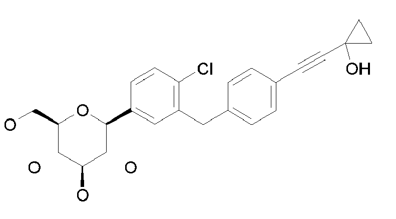 |
| 155 | 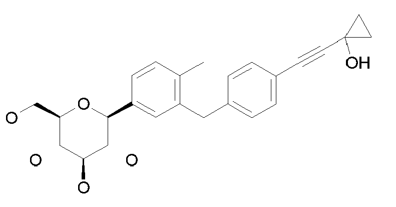 | 156 | 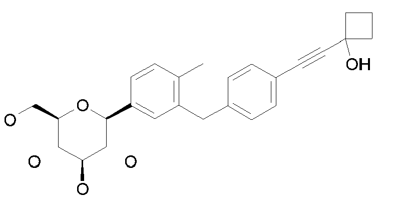 |
| 157 | 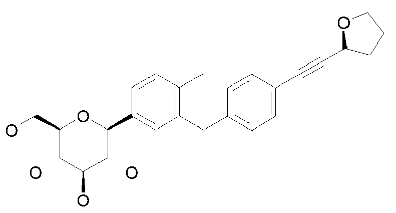 | 158 | 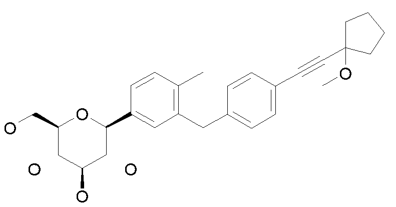 |
| 159 | 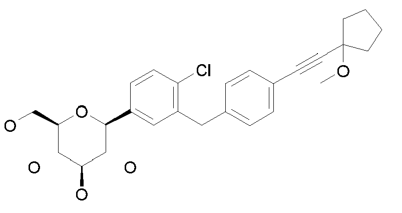 | 160 | 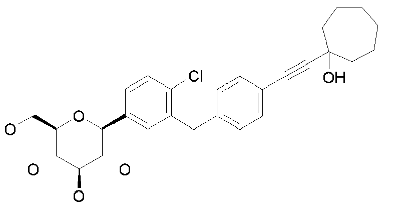 |
| 161 | 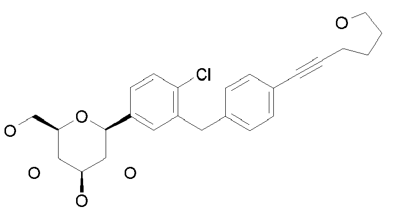 | 162 | 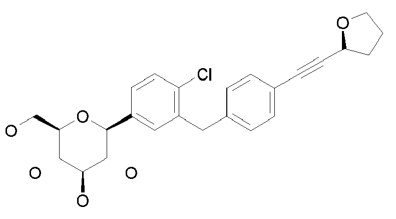 |
| 163 | 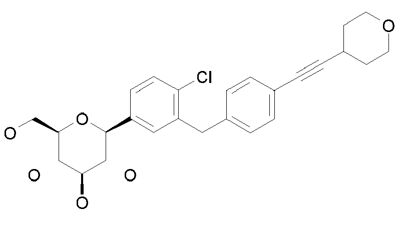 | 164 | 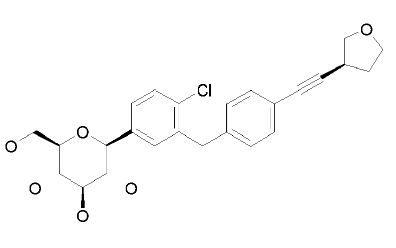 |
| 165 | 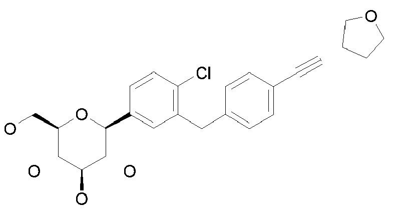 | 166 | 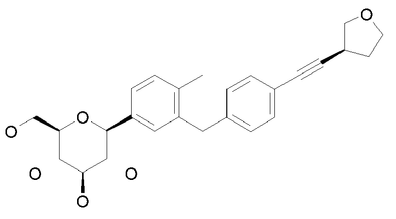 |
| 167 | 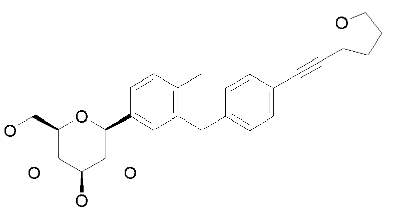 | 168 | 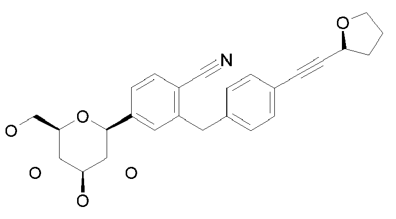 |
| 169 | 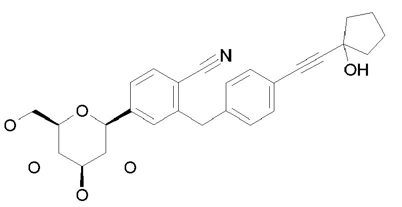 | 170 | 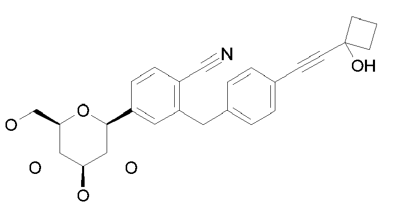 |
| 171 | 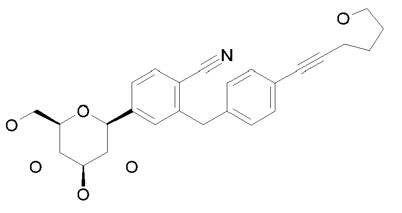 | 172 | 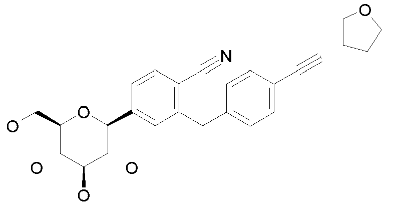 |
| 173 | 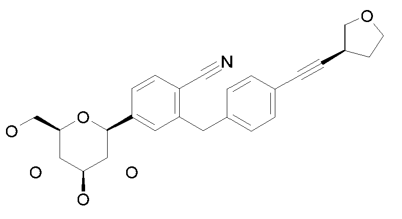 | 174 | 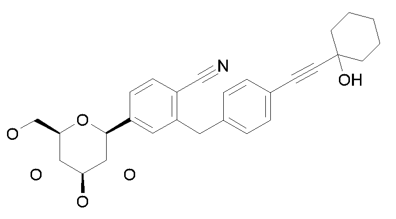 |
| 175 | 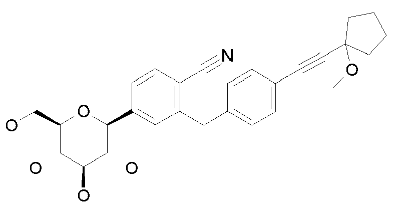 | 176 | 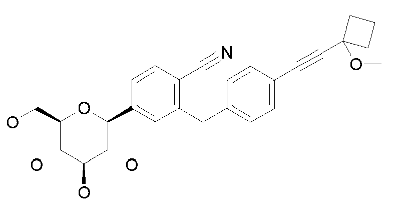 |
| 177 | 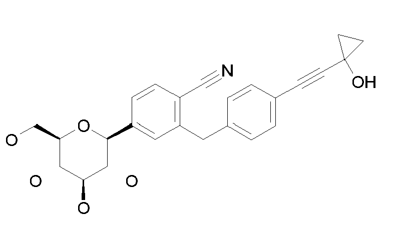 | 178 | 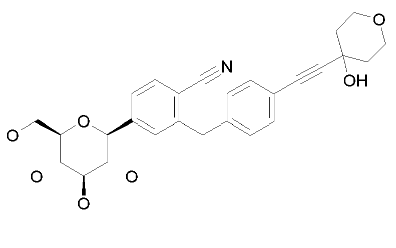 |
| 179 | 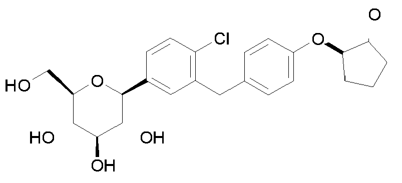 | 180 | 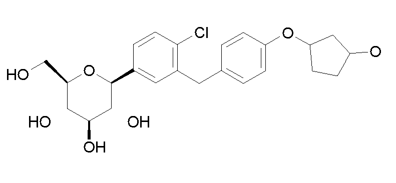 |
| 181 | 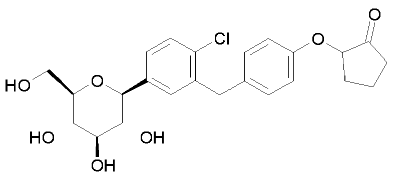 | 182 | 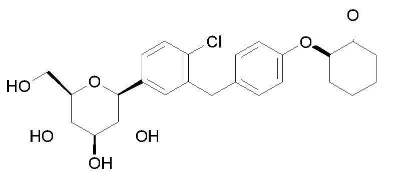 |
| 183 | 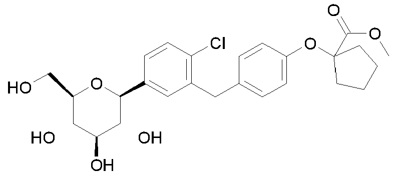 | 184 | 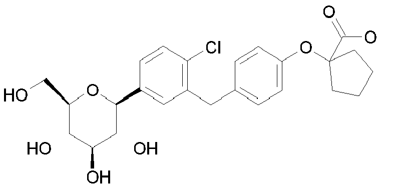 |
| 185 | 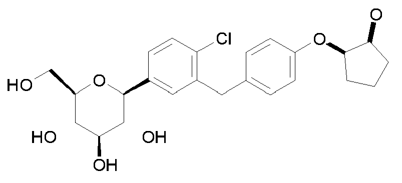 | 186 | 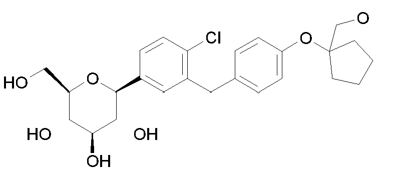 |
| 187 | 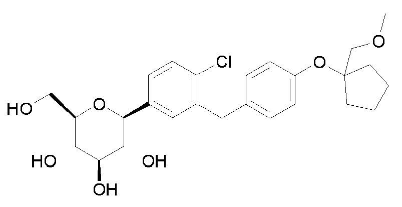 | 188 | 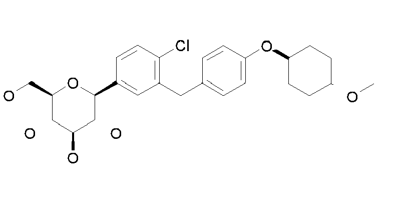 |
| 189 | 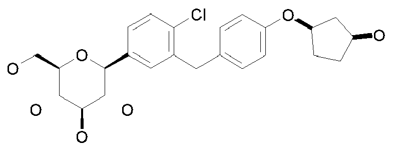 | 190 | 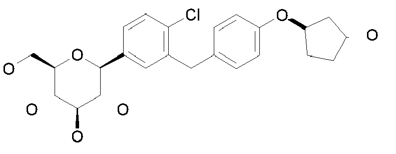 |
| 191 | 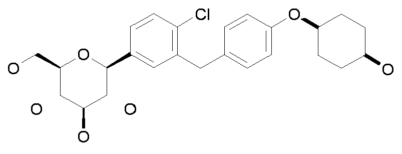 | 192 | 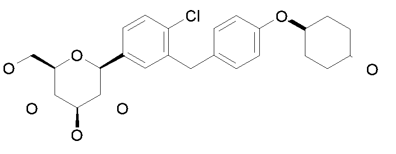 |
| 193 | 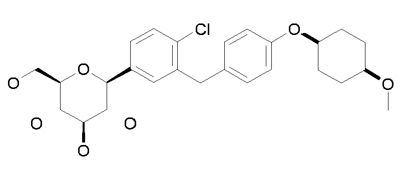 | 194 | 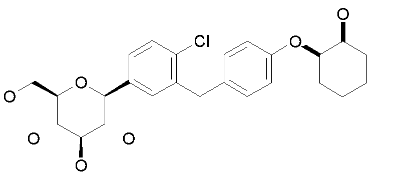 |
| 195 | 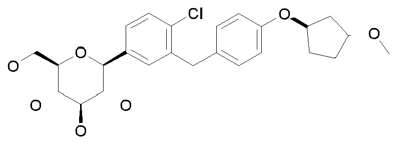 | 196 | 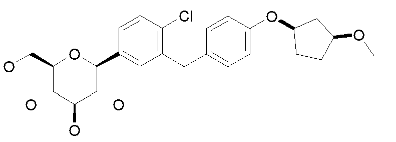 |
| 197 | 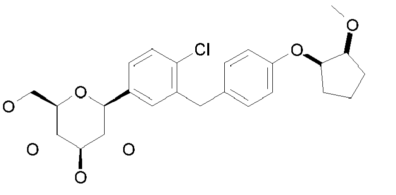 | 198 | 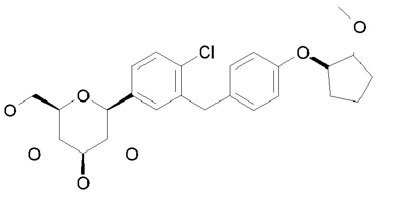 |
| 199 | 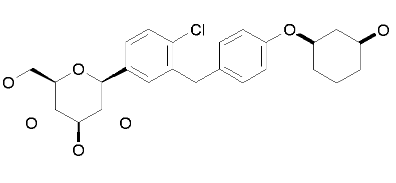 | 200 | 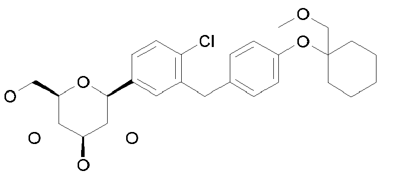 |
| 201 | 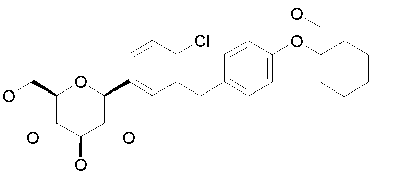 | 202 | 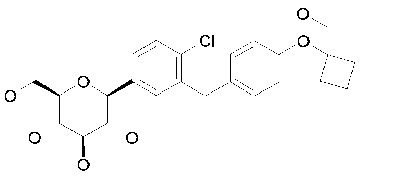 |
| 203 | 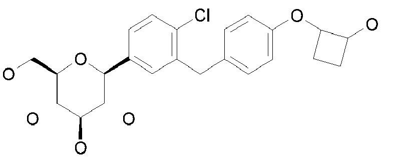 | 204 | 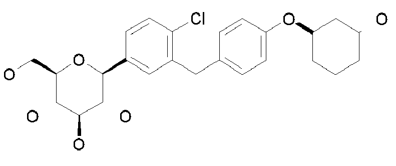 |
| 205 | 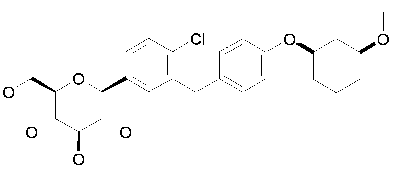 | 206 | 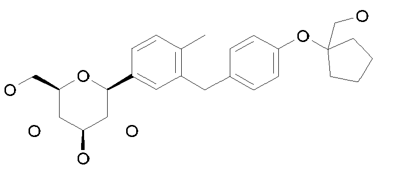 |
| 207 | 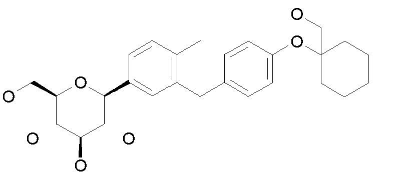 | 208 | 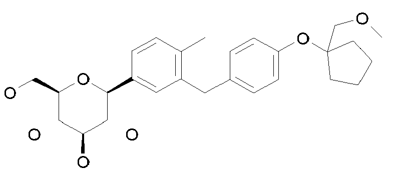 |
| 209 | 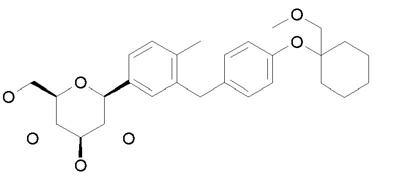 | 210 | 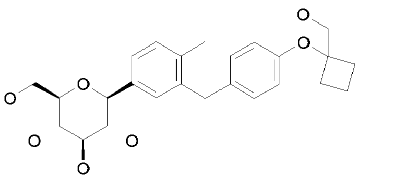 |
| 211 | 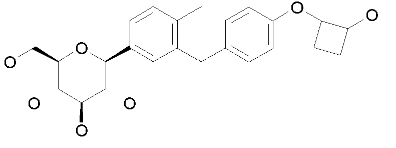 | 212 | 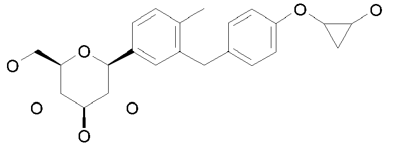 |
| 213 | 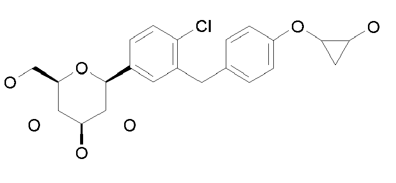 | 214 | 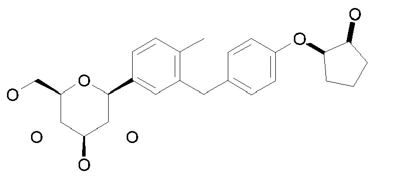 |
| 215 | 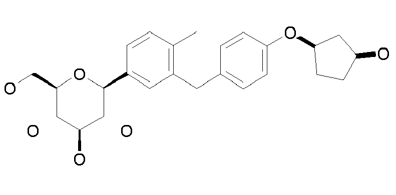 | 216 | 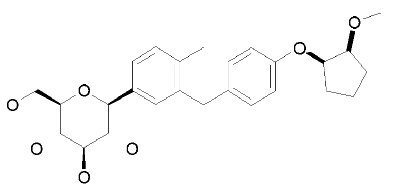 |
| 217 | 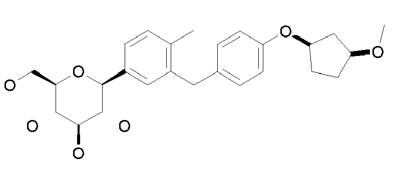 | 218 | 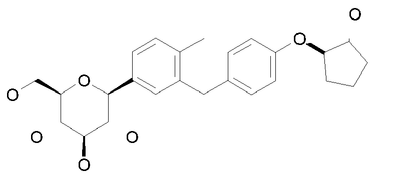 |
| 219 | 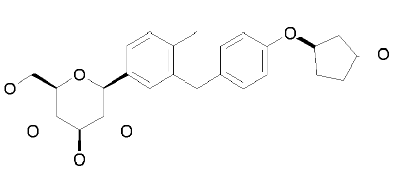 | 220 | 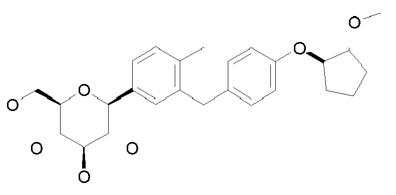 |
| 221 | 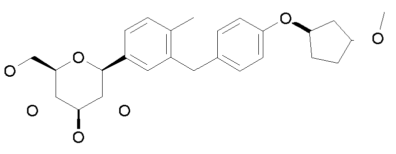 | 222 | 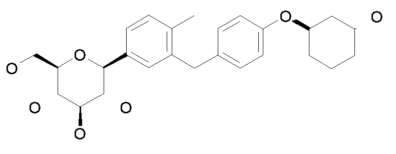 |
| 223 | 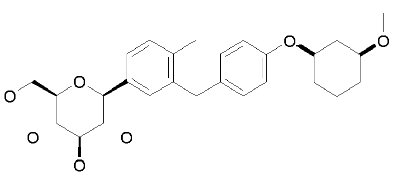 | 224 | 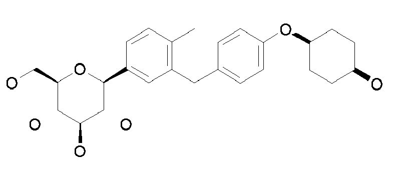 |
| 225 | 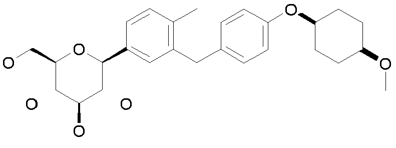 | 226 | 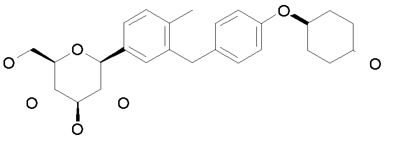 |
| 227 | 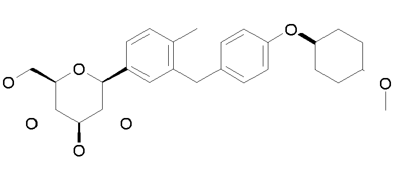 | 228 | 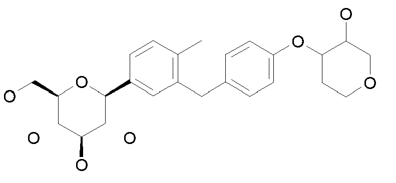 |
| 229 | 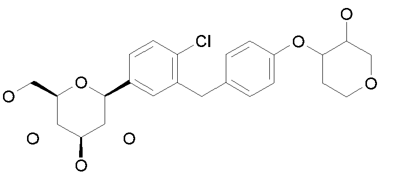 | 230 | 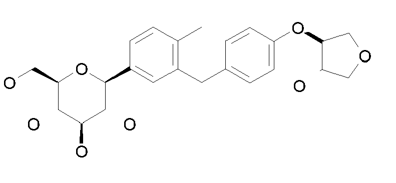 |
| 231 | 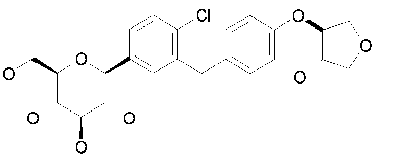 | 232 | 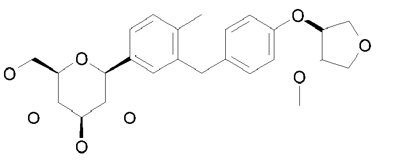 |
| 233 | 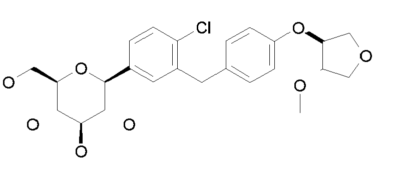 | 234 | 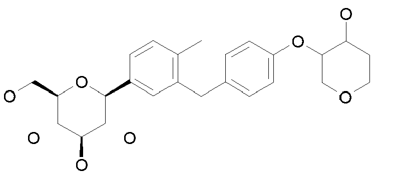 |
| 235 | 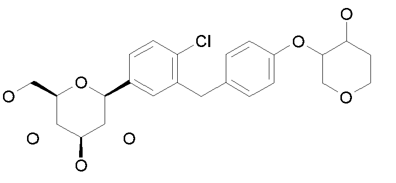 | 236 | 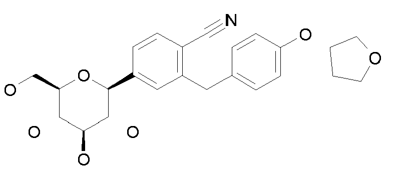 |
| 237 | 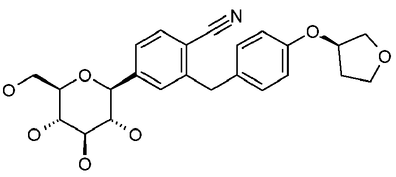 | 238 | 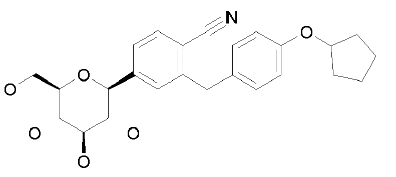 |
| 239 | 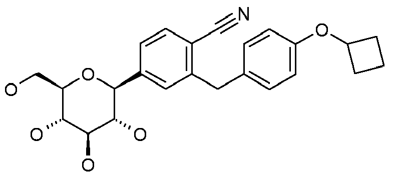 | 240 | 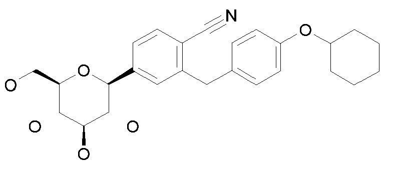 |
| 241 | 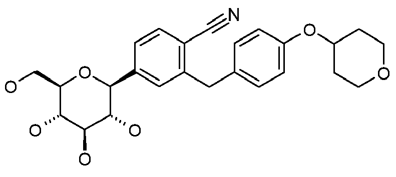 | 242 | 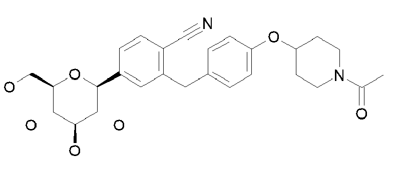 |
| 243 | 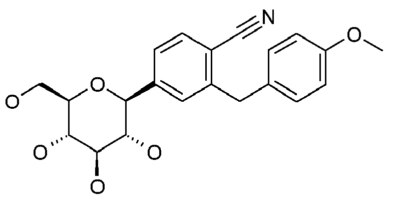 | 244 | 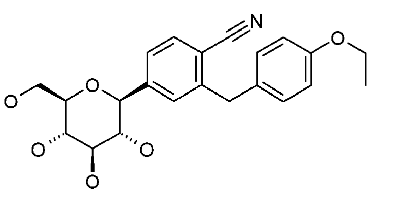 |
| 245 | 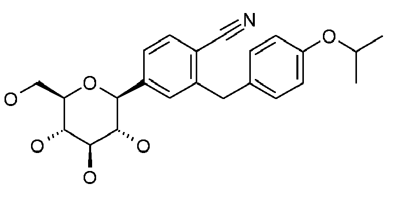 | 246 | 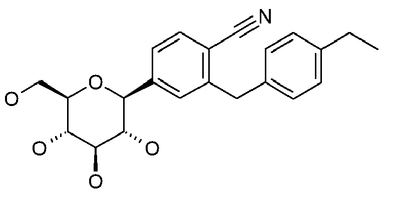 |
| 247 | 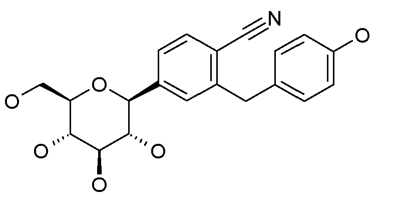 | 248 | 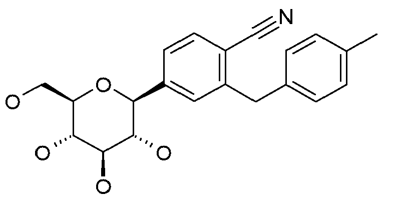 |
| 249 | 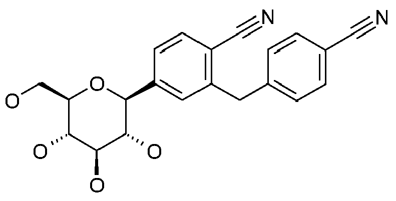 | 250 | 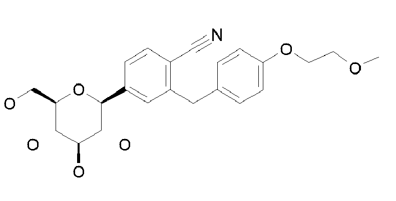 |
| 251 | 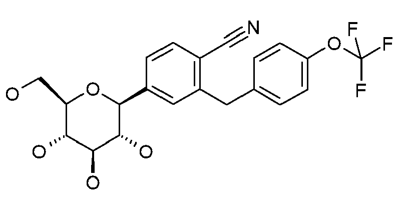 | 252 | 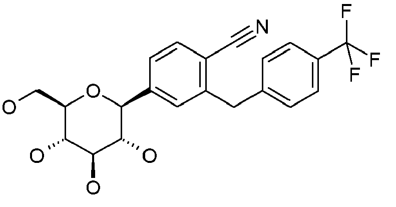 |
| 253 | 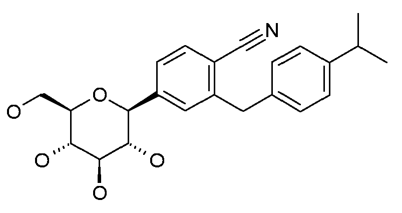 | 254 | 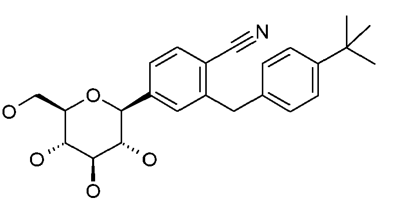 |
| 255 | 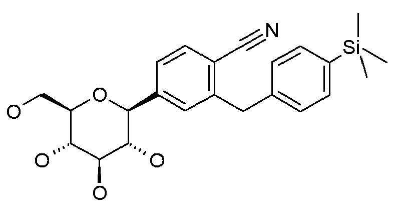 | 256 | 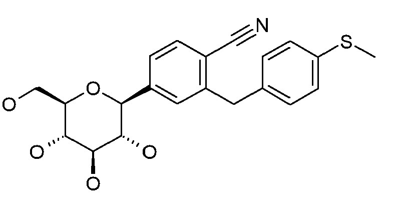 |
| 257 | 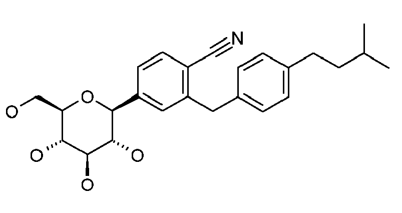 | 258 | 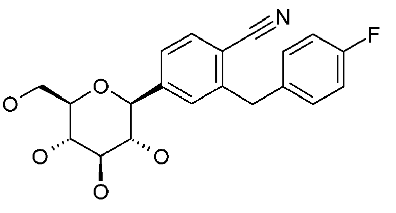 |
| 259 | 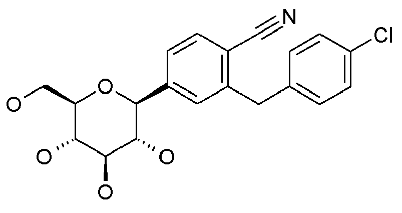 | 260 | 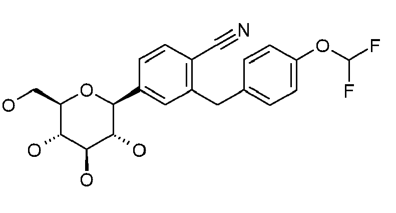 |
| 261 | 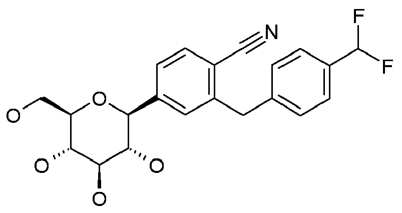 | 262 | 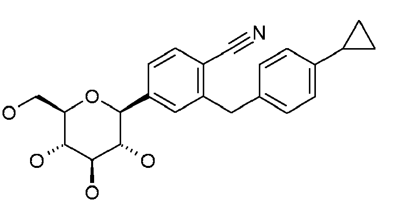 |
| 263 | 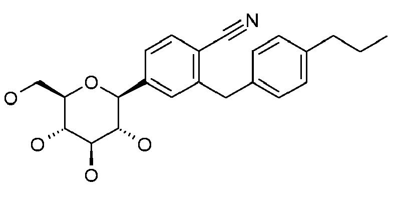 | 264 | 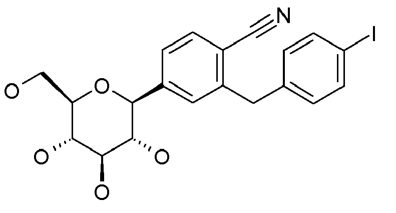 |
| 265 | 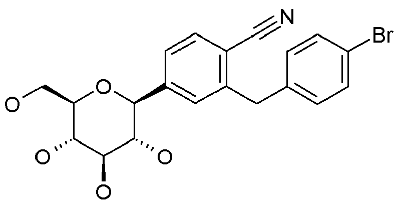 | 266 | 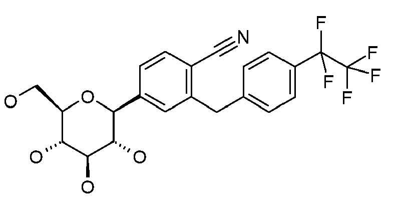 |
| 267 | 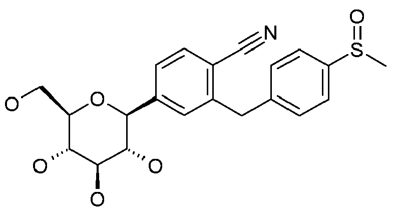 | 268 | 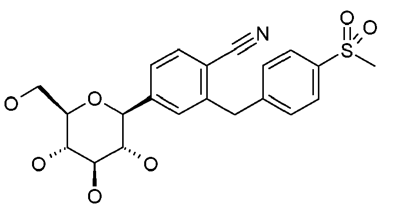 |
| 269 | 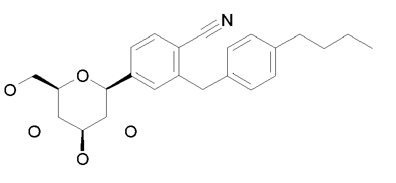 | 270 | 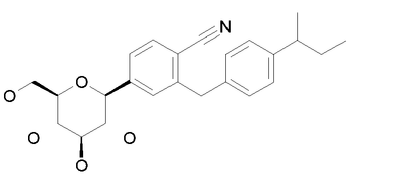 |
| 271 | 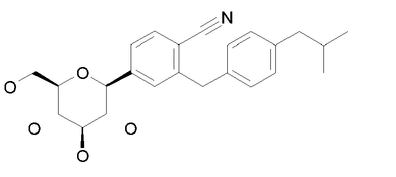 | 272 | 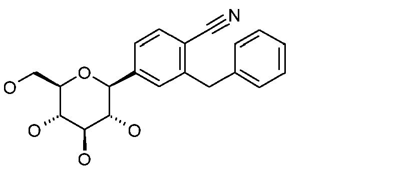 |
| 273 | 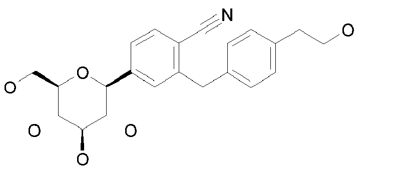 | 274 | 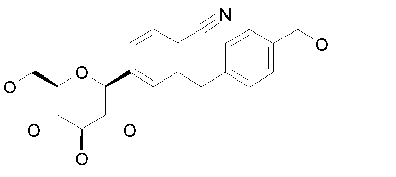 |
| 275 | 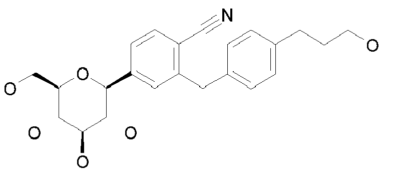 | 276 | 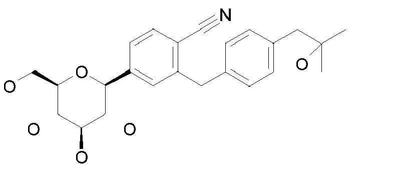 |
| 277 | 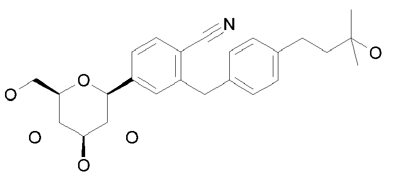 | 278 | 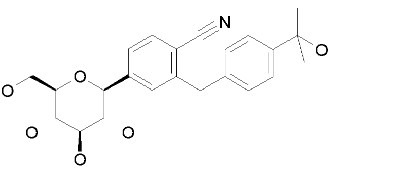 |
| 279 | 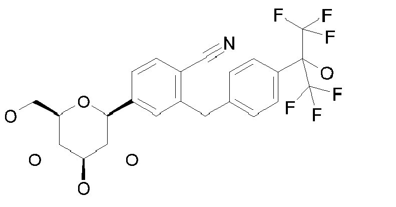 | 280 | 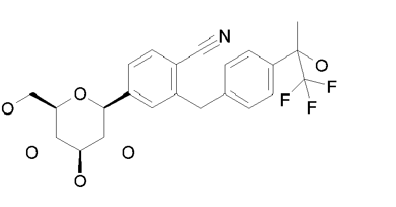 |
| 281 | 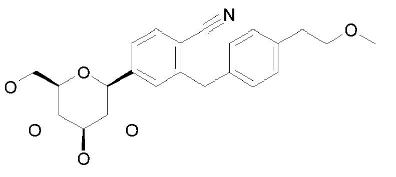 | 282 | 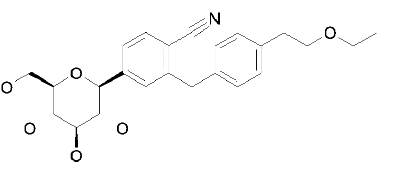 |
| 283 | 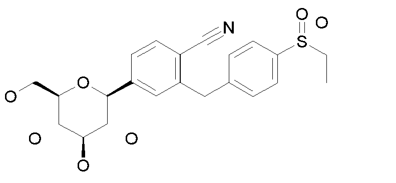 | 284 | 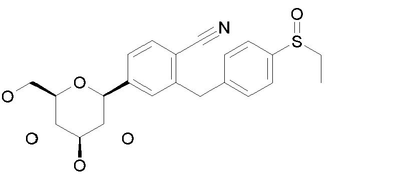 |
| 285 | 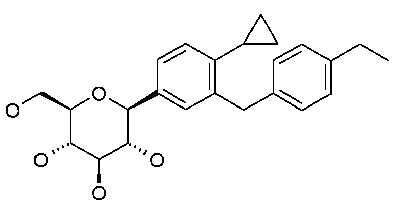 | 286 | 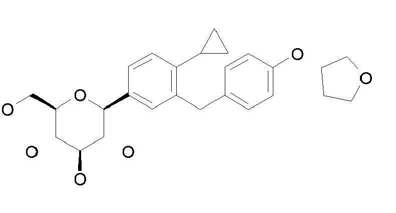 |
| 287 | 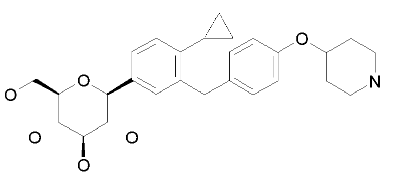 | 288 | 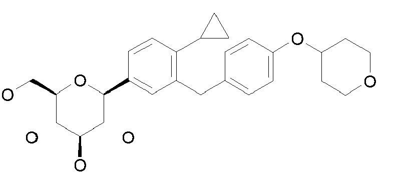 |
| 289 | 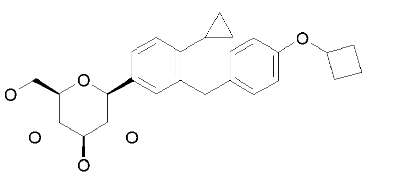 | 290 | 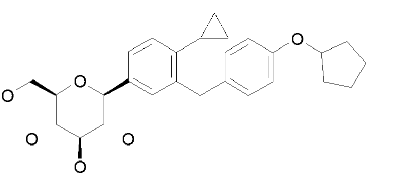 |
| 291 | 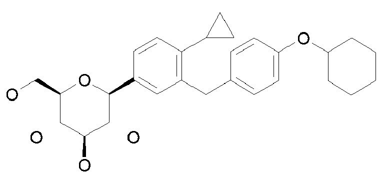 | 292 | 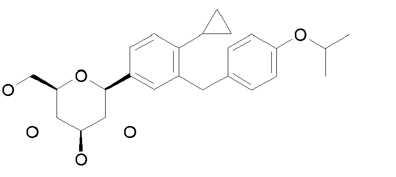 |
| 293 | 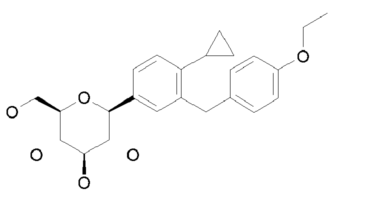 | 294 | 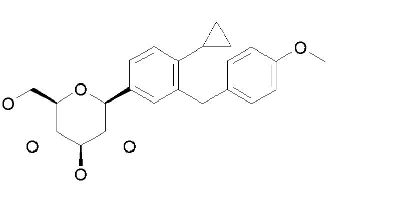 |
| 295 | 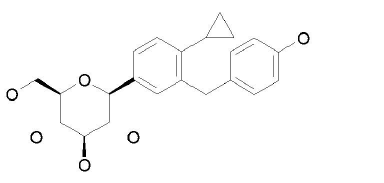 | 296 | 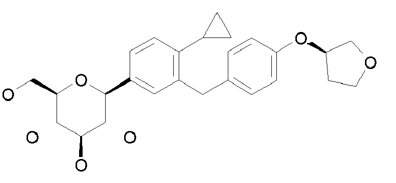 |
| 297 | 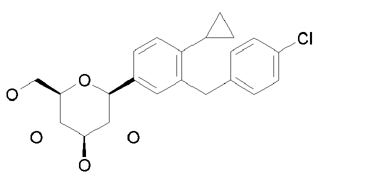 | 298 | 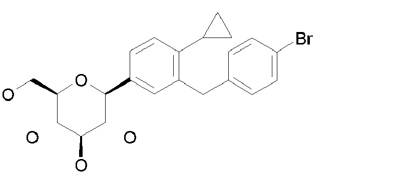 |
| 299 | 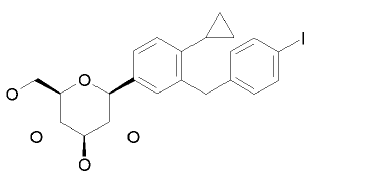 | 300 | 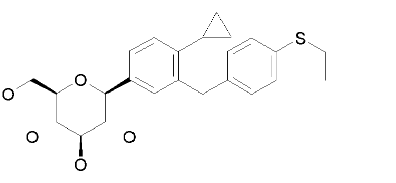 |
| 301 | 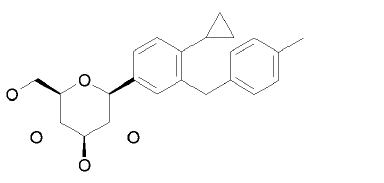 | 302 | 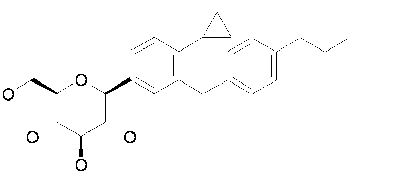 |
| 303 | 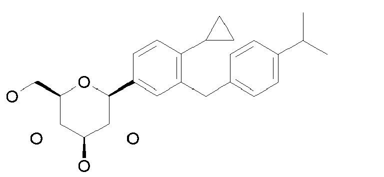 | 304 | 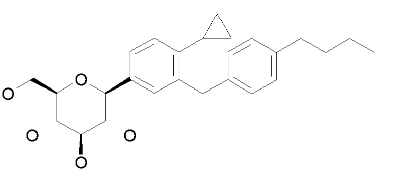 |
| 305 | 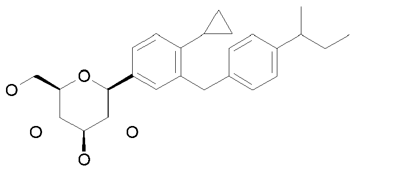 | 306 | 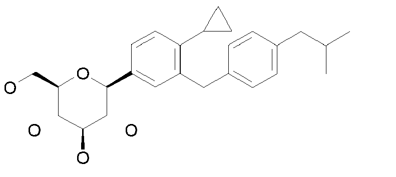 |
| 307 | 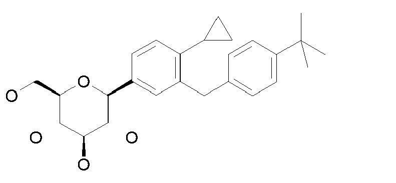 | 308 | 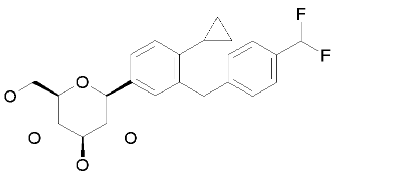 |
| 309 | 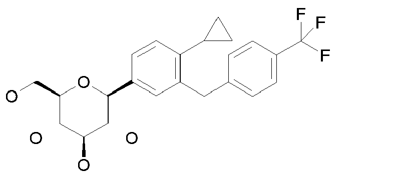 | 310 | 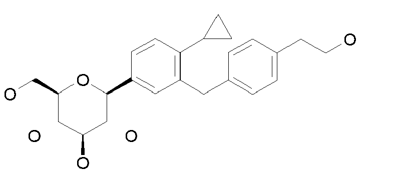 |
| 311 | 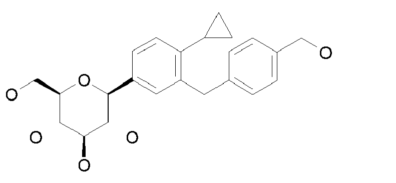 | 312 | 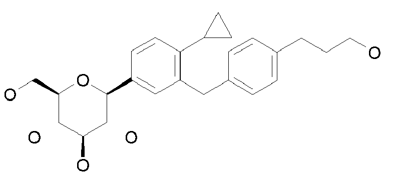 |
| 313 | 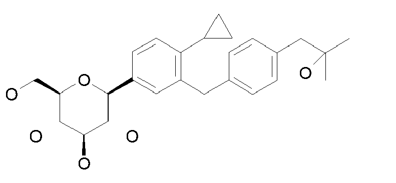 | 314 | 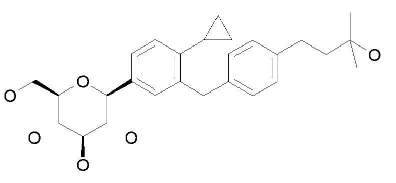 |
| 315 | 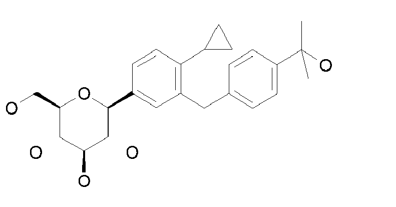 | 316 | 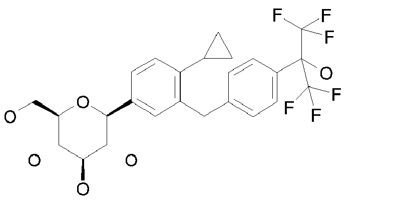 |
| 317 | 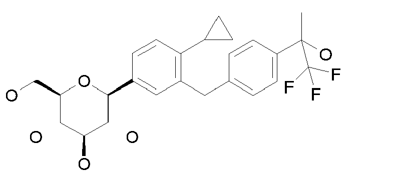 | 318 | 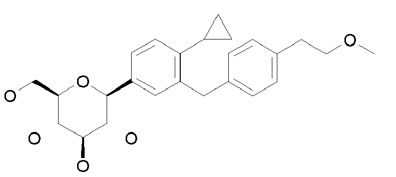 |
| 319 | 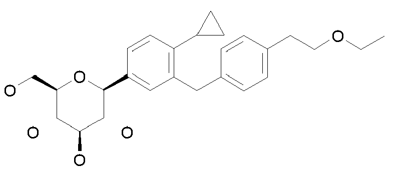 | 320 | 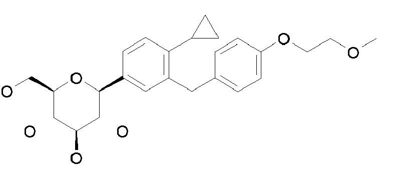 |
| 321 | 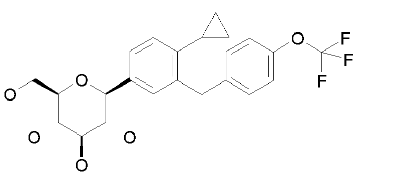 | 322 | 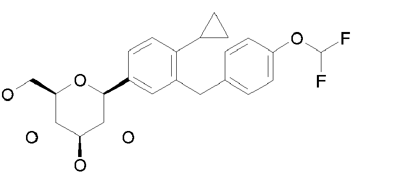 |
| 323 | 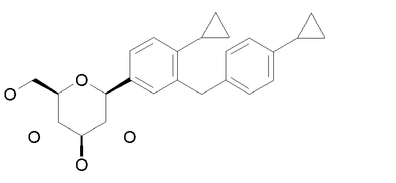 | 324 | 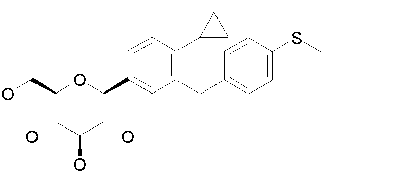 |
| 325 | 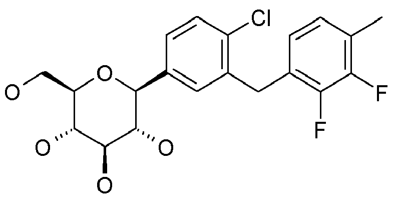 | 326 | 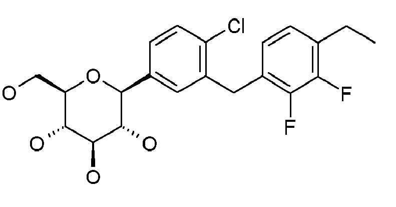 |
| 327 | 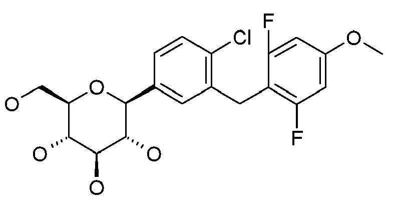 | 328 | 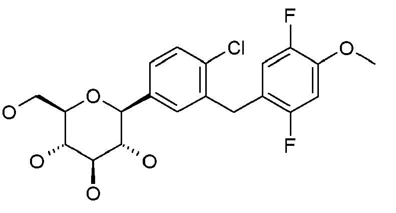 |
| 329 | 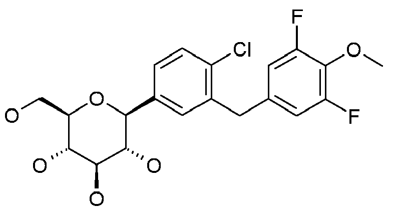 | 330 | 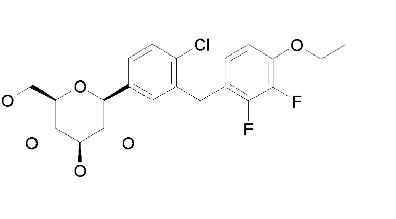 |
| 331 | 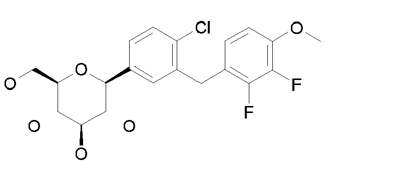 | 332 | 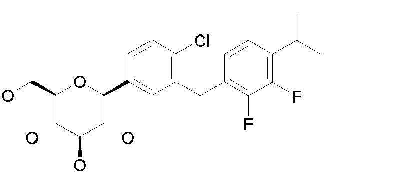 |
| 333 | 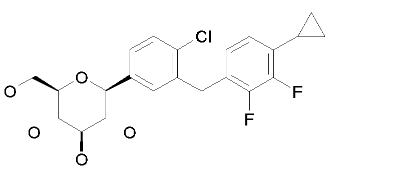 | 334 | 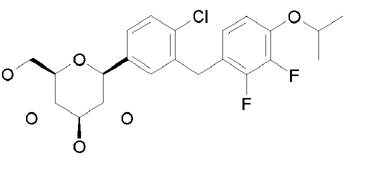 |
| 335 | 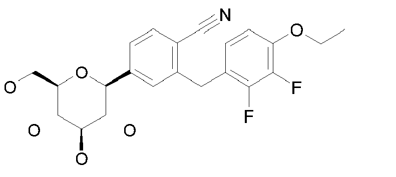 | 336 | 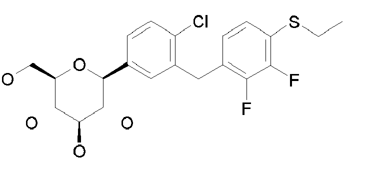 |
| 337 | 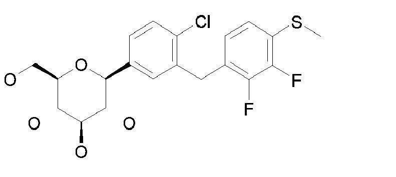 | 338 | 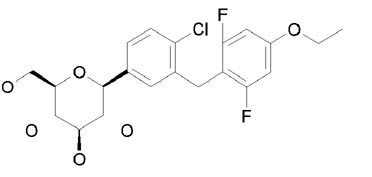 |
| 339 | 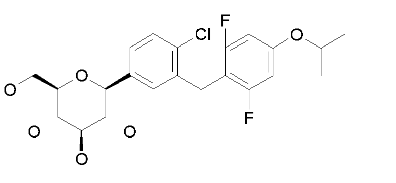 | 340 | 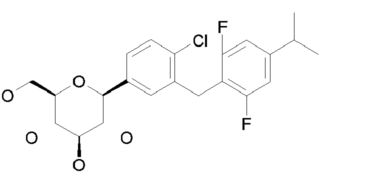 |
| 341 | 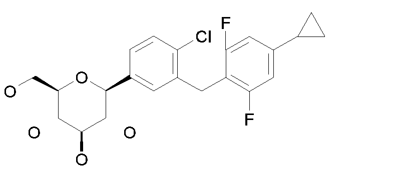 | 342 | 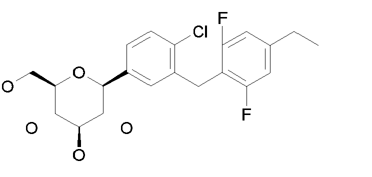 |
| 343 | 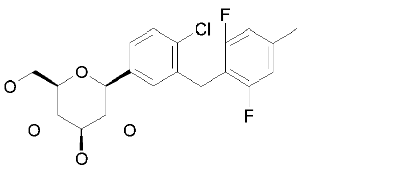 | 344 | 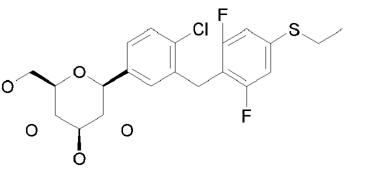 |
| 345 | 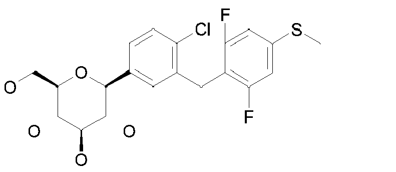 | 346 | 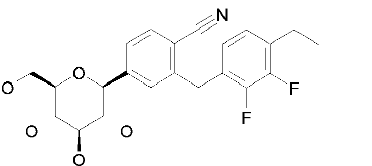 |
| 347 | 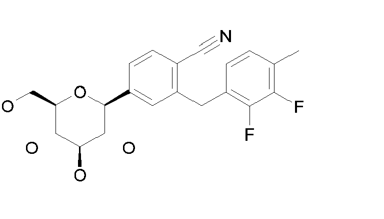 | 348 | 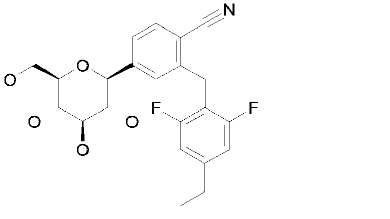 |
| 349 | 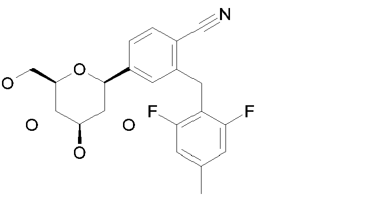 | 350 | 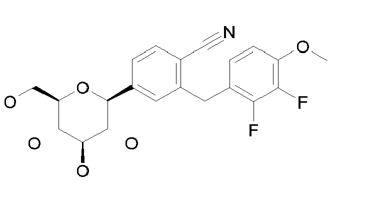 |
| 351 | 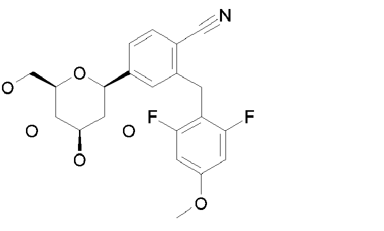 | 352 | 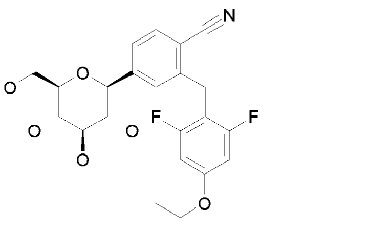 |
| 353 | 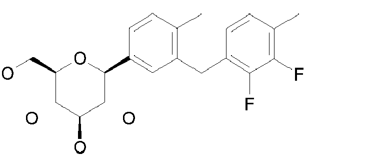 | 354 | 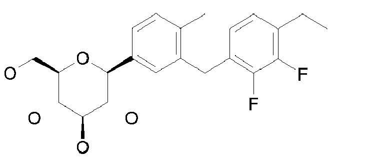 |
| 355 | 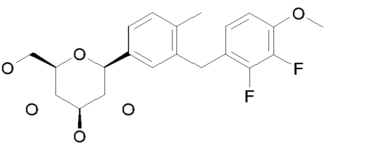 | 356 | 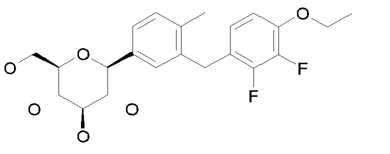 |
| 357 | 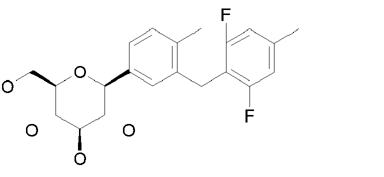 | 358 | 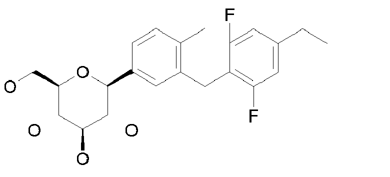 |
| 359 | 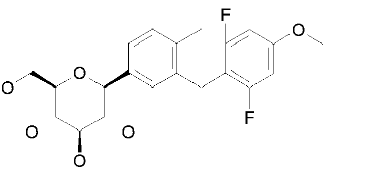 | 360 | 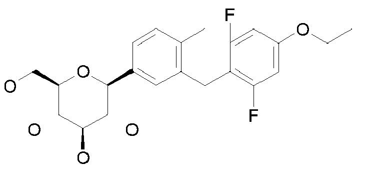 |
| 361 | 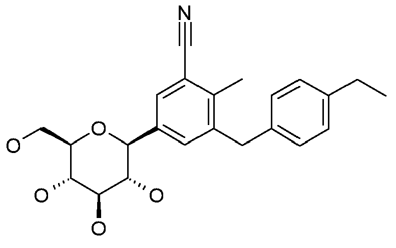 | 362 | 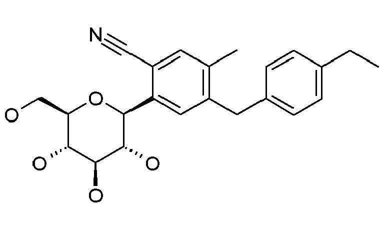 |
| 363 | 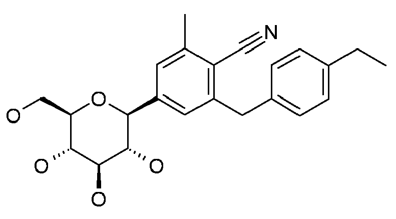 | 364 | 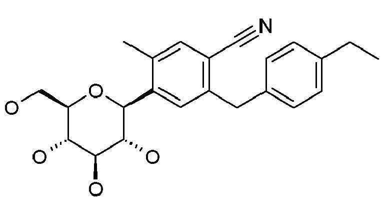 |
| 365 | 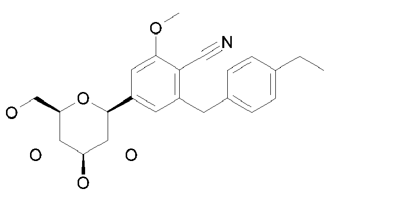 | 366 | 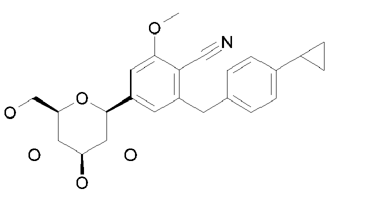 |
| 367 | 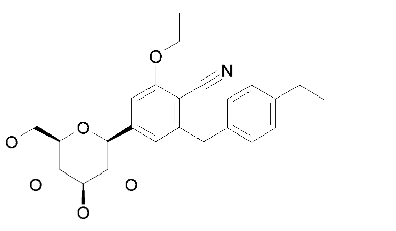 | 368 | 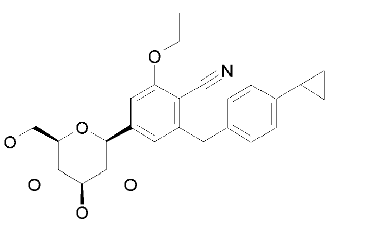 |
| 369 | 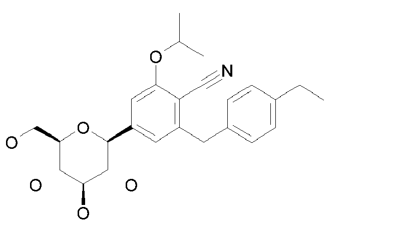 | 370 | 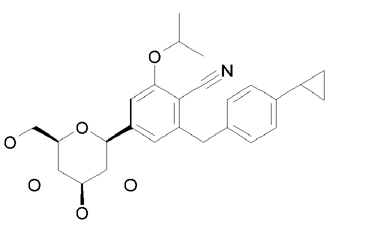 |
| 371 | 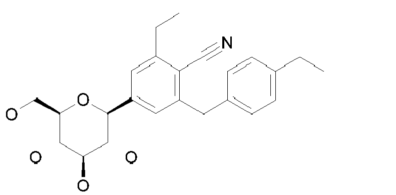 | 372 | 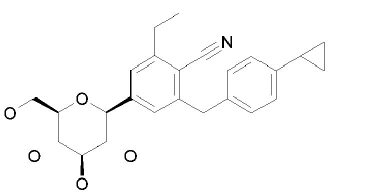 |
| 373 | 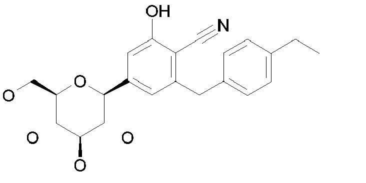 | 374 | 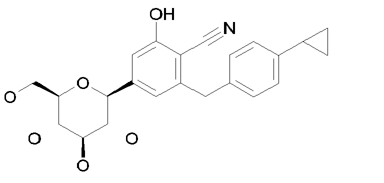 |
| 375 | 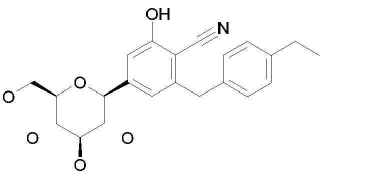 | 376 | 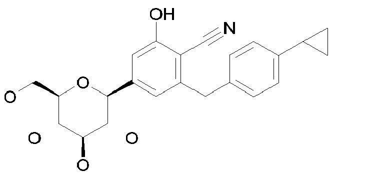 |
| 377 | 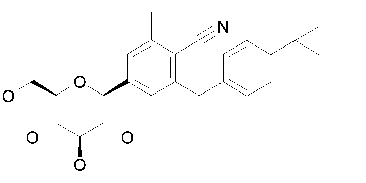 | 378 | 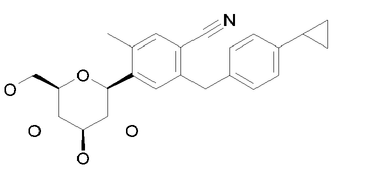 |
| 379 | 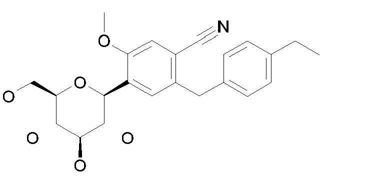 | 380 | 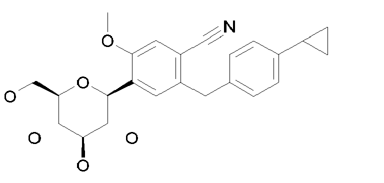 |
| 381 | 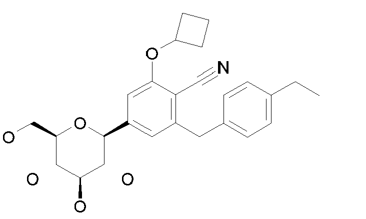 | 382 | 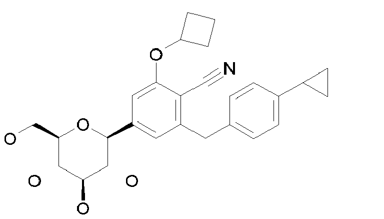 |

Some terms used above and hereinafter to describe the compounds according to the invention will now be defined more closely.

The term halogen denotes an atom selected from the group consisting of F, Cl, Br and I.

The term C₁₋ₙ-alkyl, wherein n may have a value of 2 to 18, denotes a saturated, branched or unbranched hydrocarbon group with 1 to n C atoms. Examples of such groups include methyl, ethyl, n-propyl, iso-propyl, butyl, iso-butyl, sec-butyl, tert-butyl, n-pentyl, iso-pentyl, neo-pentyl, tert-pentyl, n-hexyl, iso-hexyl, etc.

The term C₂₋ₙ-alkynyl, wherein n has a value of 3 to 6, denotes a branched or unbranched hydrocarbon group with 2 to n C atoms and a C≡C triple bond. Examples of such groups include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl etc. Unless otherwise stated alkynyl groups are connected to the remainder of the molecule via the C atom in position 1. Therefore terms such as 1-propynyl, 2-propynyl, 1-butynyl, etc. are equivalent to the terms 1-propyn-1-yl, 2-propyn-1-yl, 1-butyn-1-yl, etc.. This also applies analogously to C₂₋ₙ-alkenyl groups.

The term C₁₋ₙ-alkoxy denotes a C₁₋ₙ-alkyl-O group, wherein C₁₋ₙ-alkyl is as hereinbefore defined. Examples of such groups include methoxy, ethoxy, n-propoxy, iso-propoxy, n-butoxy, iso-butoxy, sec-butoxy, tert-butoxy, n-pentoxy, iso-pentoxy, neo-pentoxy, tert-pentoxy, n-hexoxy, iso-hexoxy etc.

The term C₁₋ₙ-alkylcarbonyl denotes a C₁₋ₙ-alkyl-C(=O) group, wherein C₁₋ₙ-alkyl is as hereinbefore defined. Examples of such groups include methylcarbonyl, ethylcarbonyl, n-propylcarbonyl, iso-propylcarbonyl, n-butylcarbonyl, iso-butylcarbonyl, sec-butylcarbonyl, tert-butylcarbonyl, n-pentylcarbonyl, iso-pentylcarbonyl, neo-pentylcarbonyl, tert-pentylcarbonyl, n-hexylcarbonyl, iso-hexylcarbonyl, etc.

The term C₃₋ₙ-cycloalkyl denotes a saturated mono-, bi-, tri- or spirocarbocyclic group with 3 to n C atoms. Examples of such groups include cyclopropyl, cyclobutyl, cyclopentyl, cyclohexyl, cycloheptyl, cyclooctyl, cyclononyl, cyclodecyl, decalinyl, bicyclo[3.2.1.]octyl, spiro[4.5]decyl, norpinyl, norbonyl, norcaryl, adamantyl, etc. Preferably the term C₃₋ₙ-cycloalkyl denotes saturated monocyclic groups.

The term C₅₋ₙ-cycloalkenyl denotes a C₅₋ₙ-cycloalkyl group which is as hereinbefore defined and additionally has at least one unsaturated C=C double bond.

The term C₃₋ₙ-cycloalkylcarbonyl denotes a C₃₋ₙ-cydoalkyl-C(=O) group wherein C₃₋ₙ-cycloalkyl is as hereinbefore defined.

The term tri-(C₁₋₄-alkyl)silyl comprises silyl groups which have identical or two or three different alkyl groups.

The term di-(C₁₋₃-alkyl)amino comprises amino groups which have identical or two different C₁₋₃-alkyl groups.

The term aryl preferably denotes naphthyl or phenyl, more preferably phenyl.

The term heteroaryl denotes a 5- or 6-membered monocyclic aromatic ring possessing one to four identical or different heteroatoms selected from the group comprising N, O and S. Heteroaryl denotes preferably a pyrrolyl, furanyl, thienyl, pyridyl or tetrazolyl group, or
a pyrrolyl, furanyl, thienyl or pyridyl group wherein one or two methine groups are replaced in each case by a nitrogen atom.

The nomenclature in structural formulas used above and hereinafter, in which a bond of a substituent of a cyclic group, as e.g. a phenyl ring, is shown towards the centre of the cyclic group, denotes, unless otherwise stated, that this substituent may be bound to any free position of the cyclic group bearing an H atom.

The compounds according to the invention may be obtained using methods of synthesis known in principle. Preferably the compounds are obtained by methods as described for example in WO 05/092877, WO 06/064033, WO 2006/120208, WO 06/089872, WO 06/108842 and in the literature cited therein.

As already mentioned, the compounds of general formula I according to the invention and the physiologically acceptable salts thereof have valuable pharmacological properties, particularly an inhibitory effect on the sodium-dependent glucose cotransporter SGLT, preferably SGLT2. In addition the compounds according to the invention of general formula I and the physiologically acceptable salts thereof are potential therapeutic agents in the treatment and/or prevention of neurodegenerative disorders, in particular dementia.

Dementia is characterized by the development of multiple cognitive deficits and memory impairment. Such cognitive deficits may include one or more of aphasia, apraxia, agnosia and disturbance in executive functioning (see for example "Diagnostic and statistical manual of mental disorders", 4th edition, American Psychiatric Association, 2000).

The compounds according to this invention are potentially valuable in the treatment of one or more neurodegenerative disorders and in preventing or slowing, delaying or reversing progression of one or more neurodegenerative disorders in a patient in need thereof.

The patient whose illness or condition is to be treated or prevented according to the invention is a mammal, particularly a human being. Preferably the term patient comprises an individual diagnosed to have a neurodegenerative disorder, in particular a dementia, especially dementia of the Alzheimer type. The term patient also comprises an individual diagnosed to have an increased risk to develop a neurodegenerative disorder, in particular a dementia, especially dementia of the Alzheimer type.

In the context of this invention the term neurodegenerative disorder denotes in particular dementia. The term dementia comprises dementia of the Alzheimer type, vascular dementia, dementia in Parkinson and dementia due to other general medical conditions. Dementia due to other medical conditions comprises dementia in chorea Huntington, dystonias, degenerative ataxias, AIDS-related dementia, Creutzfeld-Jakob's syndrome, bovine spongiform encephalopathy, prion-related infections, diseases involving mitochondrial dysfunction, Down's syndrome, hepatic encephalopathy, amyotrophic lateral sclerosis, multiple sclerosis, olivoponto-cerebellar atrophy, post-operative cognitive deficit, mild cognitive impairment, hypoxia, ischaemia resulting from cardiac arrest, stroke, glioma and other tumours, attention deficit hyperactivity disorder, autism, convulsions, epilepsy, Korsakoff syndrome, depression and schizophrenia.

The course of dementia of the Alzheimer Type is characterized by gradual onset and continuing cognitive decline.

The compounds according to this invention may improve cognitive abilities and memory, in particular in a patient as defined hereinbefore. Therefore by the administration of a compound to a patient according to this invention a cognitive decline or memory impairment may be attenuated, slowed, delayed or even reversed.

The effect of the compounds according to this invention with respect to cognitive abilities, learning and memory can be tested by methods described in the literature and known to the one skilled in the art. Examples of such tests are described in the following:
Cognitive abilities, in particular those related to learning and memorizing, may be tested in the Morris water maze. The Morris water maze is a device to investigate spatial learning and memory in rodents. It consists of a large circular pool filled with opaque water in which a small escape platform is submerged underneath the water surface. During a number of training trials, animals learn to find the platform and escape from the pool, using the different extra-maze cues contained in the experimental room. Details are described by D'Hooge R. and De Deyn P.P. (2001) "Applications of the Morris water maze in the study of learning and memory.", Brain Research Reviews 36, 60-90.

Another method to test cognitive abilities is based on contextual fear conditioning. Classical fear conditioning is a reference task to investigate fear memory. It is assessed in operant chambers where the animals receive a mild electric shock. The association between the experimental chamber and the shock is tested 24 hours later by returning the animals in the chambers in which training occurred (context) and measuring their freezing behaviour, i.e. the tendency of the animals to remain in motionless, defensive posture. Details are described by Kim J.J. and Jung M.W. (2006) "Neural circuits and mechanisms involved in Pavlovian fear conditioning: A critical review.", Neuroscience and Biobehavioral Reviews 30, 188-202.

A further test of cognitive abilities is related to the recognition of novel objects. The test is based on differential exploration of familiar and new objects. In the first trial (T1), animals are exposed to two identical objects (samples) and in a second Trial (T2), two dissimilar objects, a familiar (the sample) and a new one. Increased exploration of the novel object is a measure of recognition memory. Such a test is described by Prickaerts J. et al. (2004) "Phosphodiesterase type 5 inhibition improves early memory consolidation of object information", Neurochemistry International 45, 915-928.

The aforementioned tests of cognitive abilities can also be performed with Alzheimer disease animal models, for example with a transgenic mouse model, such as the Tg2576 mice.

The dosage required to achieve the corresponding activity for treatment or prevention usually depends on the compound which is to be administered, the patient, the nature and gravity of the illness or condition and the method and frequency of administration and is for the patient's doctor to decide. Expediently, the dosage may be from 0.1 to 100 mg, preferably 0.1 to 30 mg, by intravenous route, and 0.1 to 500 mg, preferably 0.5 to 100 mg, by oral route, in each case administered 1 to 4 times a day. For this purpose, the compounds of formula I prepared according to the invention may be formulated, optionally together with other active substances, together with one or more inert conventional carriers and/or diluents, e.g. with corn starch, lactose, glucose, microcrystalline cellulose, magnesium stearate, polyvinylpyrrolidone, citric acid, tartaric acid, water, water/ethanol, water/glycerol, water/sorbitol, water/polyethylene glycol, propylene glycol, cetylstearyl alcohol, carboxymethylcellulose or fatty substances such as hard fat or suitable mixtures thereof, to produce conventional galenic preparations such as plain or coated tablets, capsules, powders, suspensions or suppositories.

### Examples of Formulations

The following examples of formulations, which may be obtained analogously to methods known in the art, serve to illustrate the present invention more fully without restricting it to the contents of these examples. The term "active substance" denotes a glucopyranosyl-substituted benzene derivative according to this invention.

### Example 1: Dry ampoule containing 75 mg of active substance per 10 ml

| Composition: | |
|---|---|
| Active substance | 75.0 mg |
| Mannitol | 50.0 mg |
| water for injections | ad 10.0 ml |

### Preparation:

Active substance and mannitol are dissolved in water. After packaging the solution is freeze-dried. To produce the solution ready for use, the product is dissolved in water for injections.

### Example 2: Dry ampoule containing 35 mg of active substance per 2 ml

| Composition: | |
|---|---|
| Active substance | 35.0 mg |
| Mannitol | 100.0 mg |
| water for injections | ad 2.0 ml |

### Preparation:

Active substance and mannitol are dissolved in water. After packaging, the solution is freeze-dried. To produce the solution ready for use, the product is dissolved in water for injections.

### Example 3: Tablet containing 50 mg of active substance

| Composition: | |
|---|---|
| (1) Active substance | 50.0 mg |
| (2) Lactose | 98.0 mg |
| (3) Maize starch | 50.0 mg |
| (4) Polyvinylpyrrolidone | 15.0 mg |
| (5) Magnesium stearate | 2.0 mg |
| | 215.0 mg |

### Preparation:

(1), (2) and (3) are mixed together and granulated with an aqueous solution of (4). (5) is added to the dried granulated material. From this mixture tablets are pressed, biplanar, faceted on both sides and with a dividing notch on one side. Diameter of the tablets: 9 mm.

### Example 4: Tablet containing 350 mg of active substance

| Preparation: | |
|---|---|
| (1) Active substance | 350.0 mg |
| (2) Lactose | 136.0 mg |
| (3) Maize starch | 80.0 mg |
| (4) Polyvinylpyrrolidone | 30.0 mg |
| (5) Magnesium stearate | 4.0 mg |
| | 600.0 mg |

(1), (2) and (3) are mixed together and granulated with an aqueous solution of (4). (5) is added to the dried granulated material. From this mixture tablets are pressed, biplanar, faceted on both sides and with a dividing notch on one side. Diameter of the tablets: 12 mm.

### Example 5: Capsules containing 50 mg of active substance

| Composition: | |
|---|---|
| (1) Active substance | 50.0 mg |
| (2) Dried maize starch | 58.0 mg |
| (3) Powdered lactose | 50.0 mg |
| (4) Magnesium stearate | 2.0 mg |
| | 160.0 mg |

### Preparation:

(1) is triturated with (3). This trituration is added to the mixture of (2) and (4) with vigorous mixing. This powder mixture is packed into size 3 hard gelatin capsules in a capsule filling machine.

### Example 6: Capsules containing 350 mg of active substance

| Composition: | |
|---|---|
| (1) Active substance | 350.0 mg |
| (2) Dried maize starch | 46.0 mg |
| (3) Powdered lactose | 30.0 mg |
| (4) Magnesium stearate | 4.0 mg |
| | 430.0 mg |

### Preparation:

(1) is triturated with (3). This trituration is added to the mixture of (2) and (4) with vigorous mixing. This powder mixture is packed into size 0 hard gelatin capsules in a capsule filling machine.

### Example 7: Suppositories containing 100 mg of active substance

| Composition: | |
|---|---|
| Active substance | 100.0 mg |
| Polyethyleneglycol (M.W. 1500) | 600.0 mg |
| Polyethyleneglycol (M.W. 6000) | 460.0 mg |
| Polyethylenesorbitan monostearate | 840.0 mg |
| | 2,000.0 mg |

## Claims

1. Method for treating of one or more neurodegenerative disorders in a patient in need thereof wherein said method comprises administering a glucopyranosyl-substituted benzene derivative of general formula (I) 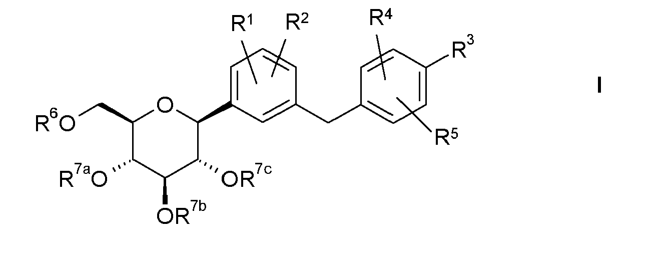 wherein
R¹ denotes hydrogen, fluorine, chlorine, bromine, iodine, cyano or nitro, or
C₁₋₄-alkyl, a methyl group substituted by 1 to 3 fluorine atoms, an ethyl group substituted by 1 to 5 fluorine atoms, a C₁₋₄-alkyl group substituted by a hydroxy or C₁₋₃-alkoxy group, or
C₂₋₆-alken-1-yl, C₂₋₄-alkenyl-C₁₋₄-alkyl, C₂₋₆-alkyn-1-yl, C₂₋₄-alkynyl-C₁₋₄-alkyl, or C₂₋₄-alkenyl-C₁₋₄-alkoxy, C₂₋₄-alkynyl-C₁₋₄-alkoxy, or
C₃₋₇-cycloalkyl, C₃₋₇-cycloalkyl-C₁₋₄-alkyl, C₅₋₇-cydoalkenyl, C₅₋₇-cycloalkenyl-C₁₋₄-alkyl, or
hydroxy, C₁₋₄-alkoxy, a methoxy group substituted by 1 to 3 fluorine atoms, an ethoxy group substituted by 1 to 5 fluorine atoms, a C₂₋₄-alkoxy group substituted by a hydroxy or C₁₋₃-alkoxy group, or
C₃₋₇-cyloalkyloxy, C₅₋₇-cydoalkenyloxy, C₃₋₆-cydoalkyl-C₁₋₃-alkoxy or C₁₋₄-alkylcarbonyl, aminocarbonyl, C₁₋₄-alkylaminocarbonyl, di-(C₁₋₃-alkyl)aminocarbonyl, pyrrolidin-1-ylcarbonyl, piperidin-1-ylcarbonyl, morpholin-4-ylcarbonyl, piperazin-1-ylcarbonyl, 4-(C₁₋₄-alkyl)piperazin-1-ylcarbonyl, C₁₋₄-alkoxycarbonyl, or
amino, C₁₋₄-alkylamino, di-(C₁₋₃-alkyl)amino, pyrrolidin-1-yl, pyrrolidin-2-on-1-yl, piperidin-1-yl, piperidin-2-on-1-yl, morpholin-4-yl, morpholin-3-on-4-yl, piperazin-1-yl, 4-(C₁₋₃-alkyl)piperazin-1-yl, C₁₋₄-alkylcarbonylamino, or
C₁₋₄-alkylsulphinyl, C₁₋₄-alkylsulphonyl, C₃₋₇-cycloalkylsulphanyl, C₃₋₇-cycloalkylsulphinyl, C₃₋₇-cycloalkylsulphonyl, C₅₋₇-cydoalkenylsulphanyl, C₅₋₇-cycloalkenylsulphinyl, C₅₋₇-cydoalkenylsulphonyl, or
aryl, heteroaryl, aryloxy, heteroaryloxy, arylcarbonyl, heteroarylcarbonyl, arylaminocarbonyl, heteroarylaminocarbonyl, aryl-C₁₋₃-alkoxycarbonyl, heteroaryl-C₁₋₃-alkoxycarbonyl, arylcarbonylamino, heteroarylcarbonylamino, arylsulphanyl, arylsulphinyl, arylsulphonyl, heteroarylsulphanyl, heteroarylsulphinyl, heteroarylsulphonyl,
while in the above-mentioned cycloalkyl and cycloalkenyl rings one or two methylene groups may be replaced independently of one another by O, S, CO, SO, SO₂ or NR^{N}, and
while the above-mentioned alkynyl and alkenyl groups may be mono- or polysubstituted by fluorine, and
the above-mentioned alkynyl and alkenyl groups may be mono- or disubstituted by identical or different groups L1, and
the above-mentioned cycloalkyl- and cycloalkenyl-rings independently of one another may be mono- or disubstituted by substituents selected from fluorine and C₁₋₃-alkyl, and
R² denotes fluorine, chlorine, bromine, iodine, hydroxy, amino, nitro, cyano, C₁₋₆-alkyl,
C₂₋₆-alkenyl, C₂₋₆-alkynyl, C₃₋₇-cycloalkyl, C₃₋₇-cycloalkyl-C₁₋₃-alkyl, C₁₋₄-alkoxy, C₃₋₇-cycloalkyloxy, C₅₋₇-cydoalkenyloxy, C₁₋₄-alkylsulfanyl, while the alkyl or alkoxy group may be mono- or polysubstituted by fluorine; and
R³ denotes hydrogen, fluorine, chlorine, bromine, iodine, cyano, nitro,
C₁₋₆-alkyl, a methyl or methoxy group substituted by 1 to 3 fluorine atoms, a C₂₋₄-alkyl or C₂₋₄-alkoxy group substituted by 1 to 5 fluorine atoms, a C₁₋₄-alkyl group substituted by a cyano group, a C₁₋₄-alkyl group substituted by a hydroxy or C₁₋₃-alkyloxy group, tri-(C₁₋₄-alkyl)silyl-C₁₋₆-alkyl,
C₂₋₆-alken-1-yl, C₂₋₄-alkenyl-C₁₋₄-alkyl,
C₂₋₆-alkyn-1-yl, C₂₋₄-alkynyl-C₁₋₄-alkyl,
C₂₋₄-alkenyl-C₁₋₄-alkoxy, C₂₋₄-alkynyl-C₁₋₄-alkoxy,
C₃₋₇-cycloalkyl, C₃₋₇-cycloalkyl-C₁₋₄-alkyl, C₅₋₇-cydoalkenyl, C₅₋₇-cycloalkenyl-C₁₋₄-alkyl, C₃₋₆-cycloalkylidenmethyl,
hydroxy, C₁₋₆-alkoxy, C₃₋₆-cycloalkyl-C₁-₃-alkoxy, C₃₋₁₀-cycloalkyloxy, C₅₋₁₀-cycloalkenyloxy, or
C₃₋₇-cycloalkylethinyl, tetrahydrofuranylethinyl, tetrahydropyranylethinyl, C₃₋₇-cycloalkyloxy, tetrahydrofuranyloxy, tetrahydropyranyloxy or cycloalkanonyl, all of which may be substituted with one to four substituents L2, or
carboxy, C₁₋₃-alkoxycarbonyl, aminocarbonyl, (C₁₋₃-alkylamino)carbonyl, di-(C₁₋₃-alkyl)aminocarbonyl, pyrrolidin-1-ylcarbonyl, piperidin-1-ylcarbonyl, morpholin-4-ylcarbonyl, piperazin-1-yl-carbonyl, 4-(C₁₋₃-alkyl)-piperazin-1-ylcarbonyl, or
amino, C₁₋₃-alkylamino, di-(C₁₋₃-alkyl)amino, pyrrolidin-1-yl, pyrrolidin-2-on-1-yl, piperidin-1-yl, piperidin-2-on-1-yl, morpholin-4-yl, morpholin-3-on-4-yl, piperazin-1-yl, 4-(C₁₋₃-alkyl)piperazin-1-yl, (C₁₋₄-alkyl)carbonylamino, C₁₋₄-alkylsulphonylamino, or
C₁₋₄-alkylsulphanyl, C₁₋₄-alkylsulphinyl, C₁₋₄-alkylsulphonyl, C₃₋₁₀-cycloalkylsulphanyl, C₃₋₁₀-cycloalkylsulphinyl, C₃₋₁₀-cycloalkylsulphonyl, C₅₋₁₀-cycloalkenylsulphanyl, C₅₋₁₀-cycloalkenylsulphinyl, C₅₋₁₀-cycloalkenylsulphonyl, or
aryl, aryl-C₁₋₃-alkyl, arylcarbonylamino, heteroarylcarbonylamino, heteroaryl, heteroaryl-C₁₋₃-alkyl, aryloxy, aryl-C₁₋₃-alkyl-oxy, arylsulphanyl, arylsulphinyl, heteroarylsulphanyl or heteroarylsulphinyl, arylsulphonylamino, aryl-C₁₋₃-alkylsulphonylamino or arylsulphonyl, or
a arylethinyl-group or a 5- or 6-membered monocyclic heteroarylethinyl-group or a 5- or 6-membered monocyclic heteroaryloxy-group;
wherein a heteroaryl-group has 1 to 4 heteroatoms independently selected from the group consisting of N, O and S; and
wherein a heteroaryl-group may possess 1 or 2 carbonyl groups as part of the monocyclic aromatic ring-system; and
wherein an N-atom of a heteroaryl ring-system may be oxidized to form the corresponding N-oxide; and
wherein one or more methine groups in a aryl- and heteroaryl-group may be substituted independently of one another with a substituent L1; and
wherein one or more imino-groups in a heteroaryl-group may be substituted independently of one another with a substituent R^{N};
while the above-mentioned alkynyl and alkenyl groups may be mono- or polysubstituted by fluorine, and
the above-mentioned alkynyl and alkenyl groups may be mono- or disubstituted by identical or different groups L1; and
while the above-mentioned cycloalkyl and cycloalkenyl rings may be mono- or disubstituted independently of one another by substituents selected from fluorine and C₁₋₃-alkyl, and
in the above-mentioned cycloalkyl and cycloalkenyl rings one or two methylene groups may be replaced independently of one another by O, S, CO, SO, SO₂ or NR^{N},
R⁴, R⁵ independently of each other denote hydrogen, fluorine, chlorine, bromine, iodine,
cyano, nitro, C₁₋₃-alkyl, C₁₋₃-alkoxy, methyl or methoxy substituted by 1 to 3 fluorine atoms, amino, C₁₋₃-alkyl-amino or di(C₁₋₃-alkyl)-amino; and
R^{N} denotes H, C₁₋₄-alkyl, C₁₋₄-alkylcarbonyl or C₁₋₄-alkylsulphonyl,
L1 independently of one another are selected from among hydroxy, cyano, nitro, C₃₋₇-cycloalkyl, C₁₋₄-alkylcarbonyl, aminocarbonyl, C₁₋₄-alkylaminocarbonyl, di-(C₁₋₃-alkyl)aminocarbonyl, C₁₋₄-alkoxycarbonyl and C₁₋₄-alkyloxy; and
L2 independently of one another are selected from among fluorine, chlorine, bromine,
iodine, C₁₋₃-alkyl, difluoromethyl, trifluoromethyl, C₁₋₃-alkoxy, difluoromethoxy, trifluoromethoxy and cyano; and
R⁶, R^{7a},
R^{7b}, R^{7c} independently of one another have a meaning selected from among hydrogen, (C₁₋₁₈-alkyl)carbonyl, (C₁₋₁₈-alkyl)oxycarbonyl, arylcarbonyl and aryl-(C₁₋₃-alkyl)-carbonyl,
while by the aryl groups mentioned in the definition of the above groups are meant phenyl or naphthyl groups which may be mono- or disubstituted independently of one another by identical or different groups L2; and
by the heteroaryl groups mentioned in the definition of the above groups are meant a pyrrolyl, furanyl, thienyl, pyridyl, indolyl, benzofuranyl, benzothiophenyl, quinolinyl, isoquinolinyl or tetrazolyl group,
or is meant a pyrrolyl, furanyl, thienyl or pyridyl group, wherein one or two methyne groups are replaced by nitrogen atoms,
or is meant an indolyl, benzofuranyl, benzothiophenyl, quinolinyl or isoquinolinyl group, wherein one to three methyne groups are replaced by nitrogen atoms,
while the above-mentioned heteroaryl groups independently of one another may be mono- or disubstituted by identical or different groups L2;
while, unless otherwise stated, the above-mentioned alkyl groups may be straight-chain or branched,
a tautomer thereof, a stereoisomer thereof, a mixture of compounds of the general formula (I) or a salt thereof,
to the patient in need thereof.

2. Method for preventing or slowing, delaying or reversing progression of one or more neurodegenerative disorders in a patient in need thereof wherein said method comprises administering a glucopyranosyl-substituted benzene derivative of general formula (I), a tautomer, stereoisomer, mixture or salt thereof, as defined in claim 1 to the patient in need thereof.

3. Method according to claim 1 or 2 wherein the neurodegenerative disorder is a dementia.

4. Method according to claim 1 or 2 wherein the neurodegenerative disorder is selected from the group consisting of dementia of the Alzheimer type, vascular dementia, dementia in Parkinson and dementia due to other general medical conditions.

5. Use of a glucopyranosyl-substituted benzene derivative of general formula (I), a tautomer, stereoisomer, mixture or salt thereof, as defined in claim 1 for the manufacture of a medicament for the treatment of one or more neurodegenerative disorders.

6. Use of a glucopyranosyl-substituted benzene derivative of general formula (I), a tautomer, stereoisomer, mixture or salt thereof, as defined in claim 1 for the manufacture of a medicament for preventing or slowing, delaying or reversing progression of one or more neurodegenerative disorders.

7. Use according to claim 5 or 6 wherein the neurodegenerative disorder is a dementia.

8. Use according to claim 5 or 6 wherein the neurodegenerative disorder is selected from the group consisting of dementia of the Alzheimer type, vascular dementia, dementia in Parkinson and dementia due to other general medical conditions.

9. Pharmaceutical composition for the treatment of one or more neurodegenerative disorders comprising a glucopyranosyl-substituted benzene derivative of general formula (I), a tautomer, stereoisomer, mixture or salt thereof, as defined in claim 1.

10. Pharmaceutical composition for preventing or slowing, delaying or reversing progression of one or more neurodegenerative disorders comprising a glucopyranosyl-substituted benzene derivative of general formula (I), a tautomer, stereoisomer, mixture or salt thereof, as defined in claim 1.
